# EUROPÄISCHE PATENTSCHRIFT

(11) **EP 3 747 983 B1**
(45) Veröffentlichungstag und Bekanntmachung des Hinweises auf die Patenterteilung: **17.08.2022**
(21) Anmeldenummer: 20178400.6
(22) Anmeldetag: 05.06.2020
(51) Int. Cl.: C12M 1/00, C12M 3/00, C12M 1/12, C12M 1/34

(54) **BIOKOMPATIBLES VERBUNDELEMENT UND VERFAHREN ZUR HERSTELLUNG EINES BIOKOMPATIBLEN VERBUNDELEMENTS**
BIOCOMPATIBLE COMPOSITE ELEMENT AND METHOD FOR PRODUCING A BIOCOMPATIBLE COMPOSITE ELEMENT
ÉLÉMENT COMPOSITE BIOCOMPATIBLE ET PROCÉDÉ DE FABRICATION D'UN ÉLÉMENT COMPOSITE BIOCOMPATIBLE

(30) Priorität: 05.06.2019 DE 102019115147
(43) Veröffentlichungstag der Anmeldung: 09.12.2020
(73) Patentinhaber: SCHOTT AG, 55122 Mainz (DE)
(72) Erfinder: OTT, Christian, 84539 Ampfing (DE); HETTLER, Robert, 84036 Kumhausen (DE); STANGL, Christoph, 84172 Buch am Erlbach (DE); BLÜMEL, Helena, 84160 Frontenhausen (DE); ECKER, Reinhard, 84032 Landshut (DE)
(74) Vertreter: Blumbach · Zinngrebe Patentanwälte PartG mbB

(56) Entgegenhaltungen:
- EP-A1- 3 553 160
- DE-A1-102008 036 934
- DE-A1-102010 007 559
- DE-A1-102013 214 422
- DE-A1-102016 008 826
- DE-A1-102017 010 629
- US-A1- 2015 108 721

## Beschreibung

Die Erfindung betrifft die spektrale Prozesskontrolle bei Produktionsprozessen in Bioreaktoren. Bioreaktoren dienen zur Kultivierung von Mikroorganismen, tierischen und pflanzlichen Zellen und eröffnen damit ein breites Anwendungsfeld von Biotech-Produktionsprozessen. Generell besteht ein Bedarf, diese Prozesse weiter zu optimieren. Insbesondere für die Herstellung von Biopharmazeutika werden Verbesserungen der Produktausbeute und damit Gewinnsteigerungen forciert. Zur Erhöhung der Produktausbeute, der Verminderung von Kontaminationsrisiken sowie der Reduktion von Kosten stehen verschiedene Ansätze zur Verfügung.

Einerseits kann die Steuerung der Prozesse und damit die Ausbeute dadurch optimiert werden, dass eine Echtzeit-Prozesskontrolle von Schlüsselparametern durchgeführt wird. Hierzu eignen sich insbesondere spektroskopische Verfahren bei denen eine Messung von außerhalb des Bioreaktors möglich ist, weil hierdurch Kontaminationsrisiken vermieden werden.

Bei einem Bioreaktor mit einem Edelstahl-Kulturgefäß können solche Messungen z.B. dadurch ermöglicht werden, dass ein Flansch-Anschlussstück mit Sichtfenster montiert wird, um ein Fenster ins Innere des Bioreaktors zu schaffen. Solche Anschlussstücke können z.B. als Metall-Glas-Verbund ausgebildet sein und bilden z.B. ein sogenanntes Glass-To-Metal-Seal (GTMS). Andererseits können Kosteneinsparungen bei Biotech-Produktionsprozessen auch dadurch erzielt werden, dass traditionelle Edelstahl-Bioreaktoren durch Einweg-Bioreaktoren (auch als Disposable-Reaktoren oder Single-Use-Reaktoren bezeichnet) ersetzt werden. Die hierbei eingesetzten Einwegmaterialen, insbesondere vorsterilisierte Kunststoffe, ermöglichen je nach Einsatzzweck deutliche Kostenreduktionen, nicht nur bei der Anschaffung, sondern auch im Betrieb, insbesondere durch Vermeidung wiederholter Reinigung und Sterilisation und der damit verbundenen Reinigungsvalidierung.

Die DE 10 2017 010 629 A1 betrifft einen ggf. als Einweg-Bioreaktor ausgebildeten Behälter mit einem Wandungsvorsprung für die Anbringung eines Sensors zur Erfassung einer Größe von einem in dem Behälter enthaltenen Medium, wobei der Wandungsvorsprung zumindest einen Sensorbereich umfasst, welcher z.B. ein Fenster umfassen kann.

Auch bei Einweg-Reaktoren hängt die Ausbeute maßgeblich von der Ermittlung von Substrat- und Produktkonzentrationen ab. Gleichwohl ist für Single-Use-Anwendungen eine in-situ Prozesskontrolle nach dem Stand der Technik noch nicht hinreichend verfügbar. Der Einsatz von Glass-To-Metal-Seals (GTMS) ist bei Single-Use-Anwendungen nicht hinreichend gegeben.

Branchenüblich werden deshalb während der Kultivierung Proben gezogen. Der Zeitaufwand ist beträchtlich. Bedingt ist dies durch den Probenzug, mit dem auch ein Kontaminationsrisiko verbunden ist, sowie durch ressourcenintensive Offline-Analytik. Trotz dieser Aufwände bleibt, insbesondere für Single-Use-Applikationen, die Prozesskontrolle durch fehlende Echtzeitdaten beeinträchtigt.

Der Erfindung liegt demnach die Aufgabe zugrunde, eine spektrale Prozesskontrolle und/oder Echtzeitdaten auch bei Einweg-Bioreaktoren bereitstellen zu können. Ein Aspekt der Aufgabe ist es, ein Anschlussstück zur Verfügung zu stellen, dass rationell insbesondere für Einweg-Bioreaktoren einsetzbar ist. Ein Aspekt der Aufgabe ist es, eine dauerhaft steril dichte und biokompatible Verbindung zu realisieren. Ein Aspekt der Aufgabe ist es demnach, eine Alternative zum Einsatz von Glas-Metall-Anschlussstücken bereitzustellen, insbesondere für Applikationen, bei denen Glass-To-Metal-Seals (GTMS) nicht präferiert sind.

Diese Aufgabe wird durch die Gegenstände der unabhängigen Ansprüche gelöst. Vorteilhafte Weiterbildungen der Erfindung sind in den abhängigen Ansprüchen definiert.

Erfindungsgemäß wird ein biokompatibles Verbundelement, insbesondere zur Verwendung als Anschlussstück für Bioreaktoren oder andere Behälter, vorzugsweise als Anschlussstück für Einweg-Bioreaktoren bereitgestellt.

Das Verbundelement umfasst eine Außenfassung mit oder aus einem Polymermaterial, insbesondere zum Anschließen an eine Wandung eines Bioreaktors, vorzugsweise zum untrennbaren Anschließen an eine Wandung eines Einweg-Bioreaktors, und eine Innenkomponente mit oder aus einem transparenten Material, z.B. Glas, insbesondere Borosikicatglas, Quarz, Saphir oder Glaskeramik. Die Innenkomponente ist steril dicht, bevorzugt hermetisch dicht in der Außenfassung aufgenommen und bildet ein Fenster, insbesondere um von außerhalb eines Bioreaktors eine spektrale Prozesskontrolle zu ermöglichen. Die Innenkomponenten ist dabei insbesondere untrennbar, also dauerhaft fest in der Außenfassung aufgenommen.

Das Verbundelement bildet insbesondere zwischen Polymerfassung und Innenkomponente eine zumindest steril dichte, bevorzugt hermetische Dichtung. Insbesondere wenn die Innenkomponente aus Glas besteht oder Glas umfasst, bietet sich die Bezeichnung Glass-To-Polymer-Seal (GTPS) an. Es handelt sich also insbesondere um Seals in Materialkombination mit Polymeren. Als Polymere für die Außenfassung, insbesondere in Form von Homo-, Co-, oder Terpolymeren, kommen insbesondere Polyethylen (PE), Polypropylen (PP), Polyamid (PA), Polyethylenterephthalat (PET), Ethylenvinyllalkohol (EVOH), Polyeterhetherketon (PEEK), Polyaryletherketon (PAEK), Polysulfon (PSU), Polyphenylensulfid (PPS)in Betracht.

Als steril dicht wird im Sinne der vorliegenden Erfindung eine Dichtung mit einer Mindestdichtwirkung verstanden. Die Dichtwirkung wird über eine Leckratenprüfung mit Helium nach dem Vakuumverfahren A3 der DIN EN 1779 bestimmt. Dabei wird ein erfindungsgemäßes Verbundelement einem zeitabhängigen Test unterzogen. Als steril dicht wird es bezeichnet, wenn die Heliumleckrate nach 2 Minuten einen Wert von weniger als 6·10⁻⁴ mbar·l/s aufweist. Eine bevorzugte Form der sterilen Dichtigkeit liegt vor, wenn die Heliumleckrate über einen Zeitraum von 2 Minuten einen Wert von weniger als 10⁻⁸ mbar·l/s aufweist. Vorteilhaft werden diese Leckraten jeweils auch bei einem Test über 4 Minuten erreicht.

Bevorzugt ist eine sogar hermetische Dichtung. Als hermetisch dicht wird eine Dichtung bezeichnet, welche eine Heliumleckrate von weniger als 1,69·10⁻¹⁰ mbar·l/s aufweist.

Das Verbundelement bzw. das Glass-To-Polymer-Seal ist insbesondere für eine universelle industrielle Nutzbarkeit ausgelegt. Im Speziellen eignet sich das Verbundelement bzw. Glass-To-Polymer-Seal für Disposable-/Single-Use-Bioreaktoren. Insbesondere für Single-Use-Bioreaktoren ermöglicht die Polymer-Außenfassung das steril dichte Verschweißen mit der Außenwandung des Bioreaktors, z.B. Polyethylen (PE) mit Polyethylen (PE). Das Polymermaterial der Außenfassung kann demnach dem Polymer des Bioreaktors entsprechen. Es sind aber auch verschiedene Materialien möglich. Das Verbundelement kann demnach dauerhaft fest mit einer Wandung eines Bioreaktors verbunden werden, so dass es nicht mehr davon getrennt werden kann.

Das erfindungsgemäße Verbundelement ist wie beschrieben biokompatibel. Verbunden damit ist insbesondere, dass bei der Kultivierung in Bioreaktoren keine unerwünschten Keime an dem Verbundelement anhaften und/oder an diesem Wachsen können. Kritisch sind insbesondere die Verbindungsbereiche zwischen Glas und Polymer und/oder Metall und Polymer. Die Biokompatibilität, d.h. insbesondere die Unterdrückung der schädlichen Keimanhaftung und/oder des schädlichen Keimwachstums kann dadurch erreicht werden, dass bei den genannten Materialübergängen keine Kantenwinkel von 90° oder mehr vorhanden sind. Damit ist selbstverständlich der Kantenwinkel im für Keime maßgeblichen mikroskopischen Maßstab gemeint, das Verbundelement kann natürlich Elemente und/oder Bereiche aufweisen, die Senkrecht zum optischen Element angeordnet sind. Die Erfindung sieht vielmehr vor, dass der mikroskopische Kantenwinkel zwischen Glas und Polymer und/oder Metall und Polymer weniger als 90° beträgt. Dies wird z.B. dadurch erreicht, dass bei der Herstellung das Polymer das Glas und/oder Metall mit einem Kantenwinkel von weniger als 90° benetzt oder dass z.B. unmittelbar nach einem Materialübergang von Metall auf Polymer bzw. von Glas auf Polymer bogenförmige Ausnehmungen im Polymermaterial eingebracht sind (siehe hierzu auch die Figuren und deren Beschreibung). Vorteilhaft kann auch ein Benetzungswinkel bzw. ein Kantenwinkel zwischen den Materialien von weniger als 85° vorgesehen sein.

Es ist demnach vorzugsweise vorgesehen, dass zumindest einer, vorzugsweise die Mehrzahl und ganz besonders bevorzugt sämtliche Kantenwinkel auf einem für Keimwachstum maßgeblichen Maßstab zwischen verschiedenen Materialien kleiner als 90°, vorzugsweise kleiner als 85° sind. Dadurch werden keimherdbegünstigende und damit der Sterilisierbarkeit abträgliche Zonen bei Übergängen verschiedener Materialien (z.B. Polymer-Glas) vermieden. Der Kontaktwinkel wird insbesondere von der polymerspezifischen Adhäsion zwischen den Makromolekülen und dem anderen Material bestimmt.

Das Prinzip der Erfindung sieht es demzufolge vor, dass ein Verbundelement zur Verfügung gestellt werden kann, das eine Außenfassung aus oder mit einem Polymermaterial aufweist und es damit rationell insbesondere für Einweg-Bioreaktoren einsetzbar macht, andererseits aber überraschend dazu in der Lage ist, eine steril dichte und biokompatible Verbindung zu realisieren, wodurch die optische Funktionalität für die Kultivierung in solchen Bioreaktoren bereitgestellt wird.

Die Erfindung ermöglicht damit allgemein eine höhere Produktausbeute und effizientere Prozessentwicklung durch eine verbesserte Prozesskontrolle. Insbesondere ermöglicht die Erfindung steril und sicher Anwendungen der spektralen in-situ Prozesskontrolle.

Für die Entwicklung und Steuerung von Biotech-Produktionsprozessen sind sterile Bedingungen und Echtzeit-Messungen (Real-Time-Messungen) von Schlüsselparametern für die Entwicklung und Steuerung von Biotech-Produktionsprozessen entscheidend.

Vorsterilisierte Single-Use-Produkte sind hierbei von besonderer Bedeutung. Sterile Kultivierungsbedingungen sind obligat, weshalb von einer Messung kein Kontaminationsrisiko ausgehen darf. Für Single-Use-Applikationen ist die branchenübliche Sterilisierbarkeitsanforderung insbesondere Gammastrahlung. In der Biotechnologie und insbesondere für die Herstellung von Biopharmazeutika sind nur zugelassene Materialen erlaubt.

Um den strengen Anforderungen für die Herstellung von Biopharmazeutika zu genügen, können die Materialien des Verbundelements so ausgewählt sein, dass dieses jeweils den nachfolgenden Standards entsprechen:
- FDA approved materials (ICH Q7A, CFR 211.65(a) - Code of Federal Regulations, USP Class, animal derivative free, bisphenol A free)
- EMA (European Medicines Agency) EU GMP Guide Part II approved materials
- Sectoral chemical resistance - ASTM D 543-06
- Biocompatibility e.g. referred to US Pharmacopeia or tests referred to ISO 10993

Die Polymer-Außenfassung des erfindungsgemäßen Verbundelements kann unterschiedlich ausgebildet sein.

Insbesondere kann die Außenfassung des Verbundelements ringförmig ausgebildet, derart, dass die Außenfassung die Innenkomponente im Querschnitt vollständig umschließt. Der Querschnitt der Innenkomponente kann dabei beliebig geformt, insbesondere rund oder kreisförmig sein.

Die Außenfassung kann außerdem rohrförmig ausgebildet sein und somit ein erstes (z.B. proximales) und ein zweites (z.B. distales) Rohrende aufweisen, insbesondere derart, dass die Außenfassung mit dem ersten Rohrende an die Wandung des Bioreaktors anschließbar ist.

Außerdem weist die Außenfassung vorzugsweise einen Flansch auf, insbesondere zum Anschließen an die Wandung eines Bioreaktors, z.B. eines Einweg-Bioreaktors. Wenn die Außenfassung rohrförmig ausgebildet ist, ist der Flansch vorzugsweise zwischen dem ersten und dem zweiten Rohrende angeordnet, kann aber auch an dem ersten Rohrende angeordnet sein.. Der Flansch kann mit der Wandung des Bioreaktors vorzugsweise dauerhaft fest, untrennbar verbunden, z.B. verschweißt werden.

Der Flansch und die Außenfassung sind vorzugsweise einstückig, monolithisch ausgebildet und insbesondere aus oder mit demselben Polymermaterial gebildet. Der Polymer-Flansch kann dann steril dicht mit der Wandung eines Polymerbehälters, insbesondere der Außenwandung eines Einweg-Bioreaktors verschweißt werden.

Ferner weist die Außenfassung vorzugsweise ein Gewinde auf, welches insbesondere als Außengewinde ausgebildet ist, wobei das Gewinde bevorzugt an dem ersten Rohrende angeordnet ist.

Auch die Innenkomponente des Verbundelements kann unterschiedlich ausgebildet sein.

Die Innenkomponente des Verbundelements ist zumindest zweiteilig ausgebildet, nämlich mit einem transparenten Bauteil und einem Verbindungsteil.

Es kann sich bei dem transparenten Bauteil beispielsweise um eine Glasscheibe handeln. Hierbei umfasst das Glas des transparenten Bauteils beispielsweise Quarzglas oder Borosilikatglas oder besteht aus diesem. Das transparente Bauteil kann aber z.B. auch als Saphirscheibe ausgebildet sein.

Vorzugsweise weist das transparente Bauteil in einem Spektralbereich mit einer Wellenlänge von 190 bis 5500 nm eine Transmission auf, die höher ist als 75%, insbesondere höher als 90 %. Bevorzugter weist das transparente Bauteil in einem Spektralbereich mit einer Wellenlänge von 190 bis 2800 nm eine Transmission auf, die höher ist als 75%, vorzugsweise höher als 80%, besonders bevorzugt höher als 90 %. Nochmals bevorzugter weist das transparente Bauteil in einem Spektralbereich n 190 bis 2700 nm eine Transmission auf, die höher ist als 75%, besonders bevorzugt höher als 90 %.

Das transparente Bauteil ist außerdem insbesondere einstückig, monolithisch ausgebildet. Wenn die Außenfassung rohrförmig ausgebildet ist, ist das transparente Bauteil bevorzugt an dem zweiten Rohrende angeordnet oder erstreckt sich im Wesentlichen bis zum zweiten Rohrende, wobei ein Rohrüberstand vorgesehen sein kann.

Wie beschrieben ist die Innenkomponente des Verbundelements zumindest zweiteilig ausgebildet, nämlich mit einem, insbesondere scheibenförmigen, transparenten Bauteil und darüber hinaus einem, insbesondere ringförmigen oder rohrförmigen Verbindungsteil.

Das Verbindungsteil ist dabei sowohl mit dem transparenten Bauteil als auch mit der Außenfassung verbunden. Die Verbindung zwischen dem Verbindungsteil und dem transparenten Bauteil ist steril dicht, vorzugsweise hermetisch dicht ausgebildet und kann auch dauerhaft fest bzw. untrennbar ausgebildet sein. Ebenso ist auch die Verbindung zwischen dem Verbindungsteil und der Außenfassung steril dicht, vorzugsweise hermetisch dicht und bevorzugt dauerhaft fest bzw. untrennbar.

Wie zuvor beschrieben kann das transparente Bauteil insbesondere einstückig, monolithisch ausgebildet sein. Ferner ist auch das Verbindungsteil vorzugsweise einstückig, monolithisch ausgebildet. Das transparente Bauteil ist bevorzugt an dem zweiten Rohrende angeordnet und das Verbindungsteil erstreckt sich bevorzugt bis zu dem ersten Rohrende.

Vorzugsweise umschließt das Verbindungsteil das transparente Bauteil im Querschnitt vollständig, derart, dass das transparente Bauteil steril und/oder hermetisch dicht, vorzugsweise dauerhaft fest bzw. untrennbar in dem Verbindungsteil aufgenommen ist und das Verbindungsteil wiederum steril und/oder hermetisch dicht, vorzugsweise dauerhaft fest bzw. untrennbar in der Außenfassung aufgenommen ist.

Es kann darüber hinaus vorgesehen sein, dass das transparente Bauteil ausschließlich mit dem Verbindungsteil in Kontakt steht. Das transparente Bauteil ist dann nur mittelbar mit der Außenfassung verbunden.

Das Verbindungsteil ist vorzugsweise mit oder aus Metall, insbesondere einem nichtrostenden Stahl, beispielsweise einem austenitisch-ferritischen Duplex-Stahl.

Es kann auch vorgesehen sein, dass das Verbindungsteil zunächst eine das transparente Bauteil umgebende Fassung, z.B. mit oder aus Glas, im Querschnitt umschließt, derart, dass das transparente Bauteil steril dicht, bevorzugt hermetisch dicht, in der Fassung aufgenommen ist, die Fassung wiederum steril dicht, bevorzugt hermetisch dicht, in dem Verbindungsteil aufgenommen ist und das Verbindungsteil wiederum steril dicht, bevorzugt hermetisch dicht, in der Außenfassung aufgenommen ist.

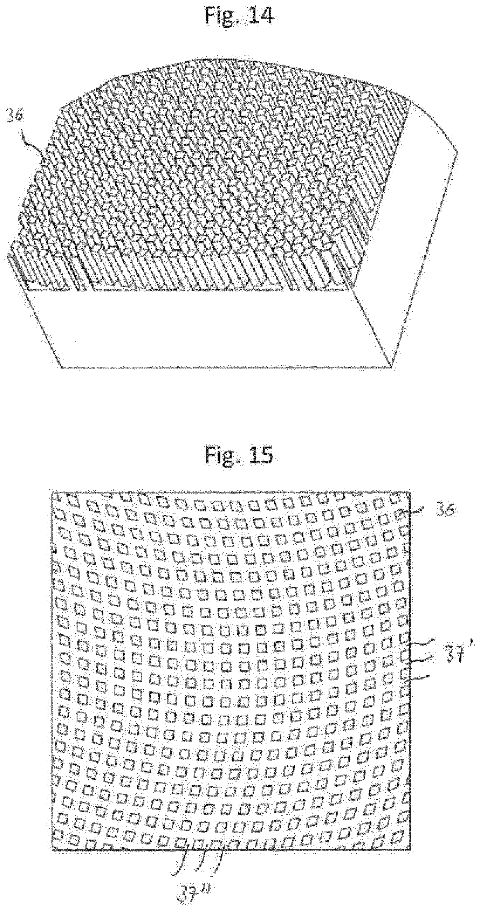

Das Verbindungsteil weist außerdem bevorzugt ein Profil zur Erhöhung der Kontaktfläche mit der Außenfassung auf. Beispielsweise können Rillen in der äußeren Oberfläche des Verbindungsteils vorgesehen sein. Im Hinblick auf die Materialverbundfestigkeit und/oder Benetzungswinkel kann insbesondere auch eine Mikrostruktur vorgesehen sein, welche z.B. durch Laserbearbeitung erzeugt sein kann. So kann die Oberfläche des Verbindungsteils etwa eine durch mehrere nebeneinander verlaufende Rillen gebildete erste Rillenschar aufweisen, welche von einer zweiten Rillenschar gekreuzt wird, so dass eine Mehrzahl oder Vielzahl von Vorsprüngen zwischen den Rillen entsteht (siehe hierzu auch Figuren 14 und 15).

Es kann außerdem alternativ oder kumulativ vorgesehen sein, dass das Verbindungsteil stirnseitig an das transparente Bauteil angrenzt und/oder stirnseitig mit dem transparenten Bauteil verbunden ist, derart, dass sowohl das transparente Bauteil als auch das Verbindungsteil jeweils steril und/oder hermetisch dicht in der Außenfassung aufgenommen sind, wobei diese Aufnahme jeweils vorzugsweise dauerhaft fest bzw. untrennbar ausgebildet ist.

Insbesondere bei einer stirnseitigen Verbindung, aber auch sonst, kann das Verbindungsteil mit oder aus einem transparenten Material, insbesondere demselben Material wie das transparente Bauteil, z.B. Glas, Saphir, Keramik oder Glaskeramik, sein. Mit anderen Worten kann das Verbundelement beispielsweise als Glas/Glas/Polymer-Verbund, als Saphir/Glas/Polymer-Verbund, als Saphir/Saphir/Polymer-Verbund, als Glas/Keramik/Polymer-Verbund, als Saphir/Keramik/Polymer-Verbund, als Keramik/Keramik/Polymerverbund, usw. ausgebildet sein. Wie weiter unten noch näher ausgeführt wird, können die aneinandergrenzenden Flächen als kohärente Flächen ausgebildet sein.

Andererseits kann das Verbindungsteil aber auch mit oder aus einem intransparentem Material sein, vorzugsweise Keramik oder Metall, insbesondere oxidierbares Metall, z.B. Aluminium, wobei besonders bevorzugt das Verbindungsteil an einer an das transparente Bauteil angrenzenden Fläche eine Oxidschicht aufweist. Die Oxidschicht kann eine Dicke von zumindest 5 Mikrometer, vorzugweise zumindest 10 Mikrometer aufweisen. Mit anderen Worten kann das Verbindungsteil an der Fügefläche in Form seines Oxides ausgeführt sein. Indem das Verbindungsteil mit oder aus einem oxidierbaren Metall gefertigt ist, kann der Fügebereich an den Schmelzpunkt des transparenten Bauteils angepasst sein, so dass Laserschweißen in Betracht kommt. Ist das Verbindungsteil z.B. aus Aluminium gefertigt (Schmelzpunkt 660°C) und an der Fügefläche eine Oxidschicht von z.B. bis zu 30 µm ausgebildet, erhöht sich Schmelzpunkt in dieser AI2O3-Zone auf 2050°C. Damit kann der Fügebereich an den Schmelzpunkt eines transparenten Bauteil z.B. aus Saphir angepasst und somit laserschweißbar sein. Für die angegebenen Temperaturwerte wird auf die GESTIS-Stoffdatenbank verwiesen.

Das Verbundelement kann Druckspannungen beherbergen. Insbesondere kann die Innenkomponente unter Druckspannung stehend in der Außenfassung aufgenommen sein.

Dies kann insbesondere dadurch erreicht werden, indem das Verbundelement wie folgt hergestellt oder herstellbar ist: Die Außenfassung wird gegenüber der Innenkomponente zum Ausdehnen gebracht (oder umgekehrt die Innenkomponente zum Zusammenziehen), danach wird die Innenkomponente, insbesondere das transparente Bauteil, in die Außenfassung eingesetzt und danach wird die Außenfassung gegenüber der Innenkomponente zum Zusammenziehen gebracht (oder umgekehrt die Innenkomponente zum Ausdehnen).

Insbesondere bei dieser Ausführungsform, aber auch unabhängig hiervon, kann die Außenfassung mit oder aus Polyetheretherketon (PEEK) sein.

Die Herstellung eines Verbundelements, welches Druckspannungen beherbergt, kann bspw. dadurch erfolgen, dass zunächst eine Außenfassung und eine Innenkomponente bereitgestellt werden, wobei die Innenkomponente gegenüber der Außenfassung ein Übermaß aufweist. Die Außenkomponente (oder die Außenfassung und die Innenkomponente) kann dann erwärmt werden, beispielsweise auf eine Temperatur von mindestens 100°C, vorzugsweise mindestens 150°C (beispielsweise 200°C) erwärmt werden, so dass sich die Außenfassung ausdehnt (oder stärker ausdehnt als die Innenkomponente) und das Übermaß der Innenkomponente verschwindet oder vorzugsweise abnimmt. Die Innenkomponente kann demnach auch weiterhin ein Übermaß aufweisen, wenn die Innenkomponente in die Außenfassung eingefügt wird. Beispielsweise kann hierbei ein Übermaß von mehr als 0,01 mm, vorzugsweise von mehr als 0,03mm (z.B. 0,04mm) vorgesehen sein. Andererseits kann ein zu großes Übermaß nicht mehr praktikabel sein, so dass das Übermaß bevorzugt geringer als 0,2mm, noch bevorzugter geringer als 0,1mm ist. Es kann demnach vorgesehen sein, dass die Innenkomponente bereits unter Erzeugung von Druckspannung in die Außenfassung eingesetzt wird. Hierbei kann eine Entstehung von Druckspannung von mehr als 10MPa, vorzugsweise mehr als 20MPa, besonders bevorzugt mehr als 30MPa vorgesehen sein. Wenn danach die Außenkomponente (bspw. auf 22°C) abkühlt, kann die Druckspannung weiter ansteigen. Beispielsweise kann vorgesehen sein, dass eine Druckspannung von mehr als 50MPa, vorzugsweise mehr als 75MPa, besonders bevorzugt mehr als 100MPa aufgebaut wird.

Unabhängig davon, ob die Innenkomponente mit oder ohne ein Übermaß in die Außenfassung eingefügt wird, ist es vorteilhaft, wenn schlussendlich die Innenkomponente unter einer Druckspannung von mindestens 50MPa, vorzugsweise mehr als 75MPa, besonders bevorzugt mehr als 100MPa in der Außenfassung aufgenommen ist, wenn das Verbundelement sich wieder unter normalen Bedingungen, insbesondere Raumtemperatur befindet. Mitunter ist die o.g. Druckspannung nicht an jeder Stelle gleich. Insbesondere, wenn die Innenkomponente an einem Rohrende einer rohrförmigen Außenfassung angeordnet ist, nimmt die Druckspannung in der Regel zum äußeren Ende hin ab (kritisches Ende). Vorzugsweise ist aber vorgesehen, dass die geringste auftretende Druckspannung (am kritischen Ende) noch größer als 1 MPa, vorzugsweise größer als 5MPa, besonders bevorzugt größer als 10MPa (beispielsweise 11 MPa) ist.

Es kann auch vorgesehen sein dass die Innenkomponente spannungsneutral in der Außenfassung aufgenommen ist.

Dies kann z.B. dadurch erreicht werden, dass das Verbundelement wie folgt hergestellt oder herstellbar ist: Die Außenfassung wird von außen an der Innenkomponente, insbesondere an dem Verbindungsteil und/oder an dem transparenten Bauteil angebracht, z.B. indem flüssiges Polymermaterial von außen an die Innenkomponente zugeführt wird und danach ausgehärtet wird.

Insbesondere bei dieser Ausführungsform, aber auch unabhängig hiervon, kann die Außenfassung mit oder aus Polyethylen (PE) sein.

Wenn das transparente Bauteil in dem Verbindungsteil aufgenommen ist, kann optional vorgesehen sein, dass das transparente Bauteil unter Druckspannung steht. Dies kommt insbesondere für den Fall eines metallischen Verbindungsteils in Betracht, aber auch allgemein.

Ein unter Druckspannung in dem Verbindungsteil aufgenommenes transparentes Bauteil kann insbesondere erreicht werden, indem die Innenkomponente wie folgt hergestellt oder herstellbar ist: Das Verbindungsteil wird gegenüber dem transparenten Bauteil zum Ausdehnen gebracht (oder umgekehrt), danach wird das transparente Bauteil in das Verbindungsteil eingesetzt und dann wird das Verbindungsteil gegenüber dem transparenten Bauteil wieder zum Zusammenziehen gebracht (oder umgekehrt).

Wenn das transparente Bauteil mit dem Verbindungsteil stirnseitig verbunden ist, aber auch allgemein, kann vorgesehen sein, dass das transparente Bauteil spannungsneutral mit dem Verbindungsteil verbunden ist. Dies kommt insbesondere für den Fall eines Verbindungsteils in Betracht, das aus oder mit dem gleichen Material wie das transparente Bauteil gefertigt ist, aber auch allgemein.

Ein spannungsneutrale Verbindung kann insbesondere dadurch erreicht werden, indem die Innenkomponente wie folgt hergestellt oder herstellbar ist: Das Verbindungsteil wird stirnseitig an das transparente Bauteil angesprengt und danach wird das Verbindungsteil vorzugsweise mit dem transparenten Bauteil fest verbunden, insbesondere laserverschweißt.

Die vorstehend beschriebenen Verfahrensabläufe werden nachfolgend nochmals detaillierter beschrieben.

Ein biokompatibles Verbundelement kann demnach z.B. gemäß folgender Verfahrensschritte, insbesondere in dieser Reihenfolge, hergestellt werden:
a) Bereitstellen einer Außenfassung mit oder aus einem Polymermaterial, insbesondere Polyetheretherketon (PEEK), und einer Innenkomponente welche zumindest zweiteilig ausgebildet ist, nämlich mit einem transparenten Bauteil, insbesondere mit oder aus Glas, Saphir oder Glaskeramik, und einem Verbindungsteil.
b) Herbeiführen eines relativen Ausdehnens der Außenfassung gegenüber der Innenkomponente, insbesondere durch Erwärmen der Außenfassung oder vorzugsweise durch gemeinsames Erwärmen der Außenfassung und der Innenkomponente.
c) Einsetzen der Innenkomponente, insbesondere des transparenten Bauteils, in die gegenüber der Innenkomponente ausgedehnten Außenfassung.
d) Herbeiführen eines relativen Zusammenziehens der Außenfassung gegenüber der Innenkomponente, insbesondere durch Abkühlen der Außenfassung oder vorzugsweise durch gemeinsames Abkühlen der Außenfassung und der Innenkomponente, so dass die Außenfassung die Innenkomponente hermetisch dicht aufnimmt.

Mit anderen Worten können in Schritt b) und entsprechend in Schritt d) die Außenfassung (oder die Außenfassung und die Innenkomponente) einer positiven bzw. negativen Wärmeausdehnung unterzogen werden, derart, dass zwischen der Außenfassung und der Innenkomponente eine temperaturbedingte Wärmeausdehnungsdifferenz erzielt wird, wobei insbesondere in Schritt b) eine positive Wärmeausdehnung (Expansion) und in Schritt d) eine negative Wärmeausdehnung (Konktraktion) ausgeführt wird.

Ein biokompatibles Verbundelement kann ferner z.B. gemäß folgender Verfahrensschritte, insbesondere in dieser Reihenfolge, hergestellt werden:
a) Bereitstellen einer Innenkomponente und flüssigen Polymermaterials zur Bildung einer Außenfassung.
b) Zuführen des flüssigen Polymermaterials von außen an die Innenkomponente, insbesondere an das Verbindungsteil und/oder an das transparente Bauteil.
c) Aushärten des flüssigen Polymermaterials, derart, dass eine die Innenkomponente steril dicht, bevorzugt hermetisch dicht aufnehmende Außenfassung gebildet wird.

Um in den Schritten a) die Innenkomponente bereitzustellen, können den vorstehend beschriebenen Verfahrensabläufen noch die folgenden Verfahrensschritte, insbesondere in dieser Reihenfolge, vorangehen:
aa) Bereitstellen eines transparenten Bauteils und eines Verbindungsteils, insbesondere aus Metall.
bb) Herbeiführen eines relativen Ausdehnens des Verbindungsteils gegenüber dem transparenten Bauteil, insbesondere durch Erwärmen des Verbindungsteils oder vorzugsweise durch gemeinsames Erwärmen des Verbindungsteils und des transparenten Bauteils.
cc) Einsetzen des transparenten Bauteils in das gegenüber dem transparenten Bauteil ausgedehnte Verbindungsteil.
dd) Herbeiführen eines relativen Zusammenziehens des Verbindungsteils gegenüber dem transparenten Bauteil, insbesondere durch Abkühlen des Verbindungsteils oder vorzugsweise durch gemeinsames Abkühlen des Verbindungsteils des transparenten Bauteils, so dass sich das Verbindungsteil mit dem transparente Bauteil zu der Innenkomponente insbesondere steril dicht und/oder hermetisch dicht verbindet.

Mit anderen Worten können in Schritt bb) und entsprechend in Schritt dd) das Verbindungsteil (oder das Verbindungsteil und das transparente Bauteil) einer positiven bzw. negativen Wärmeausdehnung unterzogen werden, derart, dass zwischen dem Verbindungsteil und dem transparenten Bauteil eine temperaturbedingte Wärmeausdehnungsdifferenz erzielt wird, wobei insbesondere in Schritt bb) eine positive Wärmeausdehnung (Expansion) und in Schritt dd) eine negative Wärmeausdehnung (Kontraktion) ausgeführt wird.

Um in den Schritten a) die Innenkomponente bereitzustellen, können andererseits auch die folgenden Verfahrensschritte, insbesondere in dieser Reihenfolge, vorangehen:
aa) Bereitstellen eines transparenten Bauteils und eines Verbindungsteils, insbesondere aus demselben Material wie das transparente Bauteil.
bb) Heranführen und/oder Ansprengen des Verbindungsteils stirnseitig an das transparente Bauteil, so dass sich das Verbindungsteil mit dem transparente Bauteil zu der Innenkomponente insbesondere steril dicht und/oder hermetisch dicht verbindet.
cc) Vorzugsweise Laserverschweißen der beiden Bauteile, so dass sich das Verbindungsteil mit dem transparente Bauteil zu der Innenkomponente insbesondere steril dicht und/oder hermetisch dicht und fest verbindet.

Sämtliche Verfahrensabläufen zur Herstellung eines biokompatiblen Verbundelements umfassen ferner vorzugsweise, insbesondere als abschließenden Schritt, das Sterilisieren, insbesondere Autoklavieren, des Verbundelements umfassend die Außenfassung und die darin steril dicht und/oder hermetisch dicht aufgenommene Innenkomponente.

Die Erfindung betrifft außerdem einen Bioreaktor, insbesondere einen Einweg-Bioreaktor, zur Kultivierung von Mikroorgansimen oder tierischen oder pflanzlichen Zellen mit einem an eine Wandung des Bioreaktors als Anschlussstück angeschlossenen, z.B. Polymer-zu-Polymer verschweißten, Verbundelements wie vorstehend beschrieben.

An dem Bioreaktor, insbesondere dem Einweg-Bioreaktor, ist an der Wandung als Anschlussstück das Verbundelement insbesondere dauerhaft fest angeschlossen, z.B. angeschweißt. Das Verbundelement bzw. das Anschlussstück kann insbesondere die vorstehend beschriebenen Merkmale aufweisen. Die Innenkomponente des Verbundelements ist zumindest zweiteilig ausgebildet, nämlich mit einem transparenten Bauteil und einem Verbindungsteil.

Insbesondere weist das Verbundelement eine Außenfassung mit oder aus einem Polymermaterial auf, wobei die Außenfassung an der Wandung des Bioreaktors angeschlossen ist, insbesondere damit steril dicht und/oder hermetisch dicht verbunden ist. Ferner weist das Verbundelement eine Innenkomponente mit oder aus einem transparenten Material, z.B. Glas oder Glaskeramik, auf, wobei die Innenkomponente steril dicht und/oder hermetisch dicht in der Außenfassung, insbesondere dauerhaft fest, aufgenommen ist und ein Fenster in den Bioreaktor bildet, insbesondere um von außerhalb des Bioreaktors eine spektrale Prozesskontrolle zu ermöglichen.

Die Außenfassung ist vorzugsweise rohrförmig mit einem ersten und einem zweiten Rohrende ausgebildet und an der Wandung des Bioreaktors angeschlossen, z.B. mit dem ersten Rohrende an der Wandung des Bioreaktors angeschlossen. Ferner weist die Außenfassung vorzugsweise einen Flansch auf und ist mit dem Flansch an der Wandung des Bioreaktors angeschlossen, wobei der Flansch bevorzugt zwischen dem ersten und dem zweiten Rohrende angeordnet ist oder an dem ersten Rohrende angeordnet ist.

Der insbesondere als Einweg-Bioreaktor ausgebildete Bioreaktor, welcher insbesondere zur Aufnahme von fluiden Medien, die biologisches Material enthalten, ausgebildet ist, weist vorzugsweise einen Kunststoff, insbesondere einen sterilisierbaren Kunststoff auf oder besteht aus einem Kunststoff, insbesondere einem sterilisierbaren Kunststoff.

Der Bioreaktor ist vorzugsweise zusammen mit dem daran angeschlossenen Verbundelement autoklavierbar.

Der Bioreaktor mit einem an eine Wandung des Bioreaktors als Anschlussstück angeschlossenen Verbundelement weist vorzugsweise hinsichtlich des Verbundelements die Merkmale eines der Ansprüche 1 bis 11 auf.

Schließlich betrifft die Erfindung auch ein Verfahren zur Vermehrung oder Kultivierung biologischen Materials, insbesondere von Mikroorgansimen oder Zellen, in einem Bioreaktor, insbesondere einem Einweg-Bioreaktor, wie vorstehend beschrieben, mit einem an eine Wandung des Bioreaktors als Anschlussstück angeschlossenen biokompatiblen Verbundelement nach einem der Ansprüche 1 bis 11, wobei von außerhalb des Bioreaktors durch das gebildete Fenster eine spektrale Prozesskontrolle erfolgt, wobei insbesondere eine physikalische, chemische oder biologische Messgröße von außerhalb des Bioreaktors durch das gebildete Fenster erfasst wird.

Ein bevorzugtes Verfahren zur Vermehrung oder Kultivierung biologischen Materials umfasst das Einbringen fluiden, insbesondere biologischen Materials oder einer Vorstufe biologischen Materials in einen Bioreaktor, wie dieser hier beschrieben ist, sowie das Erfassen einer physikalischen, chemischen oder biologischen Messgröße unter Verwendung eines Fensters gebildet durch ein Anschlussstück wie vorstehend beschrieben.

Vorteilhaft können die hier offenbarten Verfahren zur Vermehrung oder Kultivierung die Herstellung von Pharmazeutika, insbesondere von Biopharmazeutika umfassen.

Bevorzugt wird der Bioreaktor nach dem, insbesondere untrennbaren, Anschließen des Anschlussstücks sterilisiert oder autoklaviert wird. Es kann dann das Erfassen von Messwerten von außerhalb des Bioreaktors erfolgen. Vorzugsweise kann eine ortsaufgelöste Messung erfolgen, um beispielsweise Stoffwechselvorgängen ortsaufgelöst zu erfassen.

Das Verfahren zur Vermehrung oder Kultivierung biologischen Materials kann umfassen, dass von außerhalb des Bioreaktors die Strahlungsintensität und/oder die Wellenlänge der elektromagnetischen Strahlung im Innern des Bioreaktors, insbesondere ortsaufgelöst, gemessen wird.

Wenn für einen definierten Zeitraum von außerhalb des Bioreaktors elektromagnetische Strahlung einer definierten Wellenlänge, bevorzugt 250 nm in den Bioreaktor eingestrahlt wird und nach dieser Einstrahlung breitbandig oder selektiv bei einer Wellenlänge, insbesondere bei 270 nm eine Strahlungsintensität und/oder Wellenlänge der elektromagnetischen Strahlung im Innern des Bioreaktors gemessen wird, können hierdurch fluoreszent emittierte Anteile des Lichts detektiert und bezüglich definierter Stoffwechselvorgänge innerhalb des Photobioreaktors ausgewertet werden.

Demnach kann z.B. vorgesehen sein, dass von außerhalb des Bioreaktors für einen definierten Zeitraum elektromagnetische Strahlung einer definierten Wellenlänge, bevorzugt 250 nm in den Bioreaktor eingestrahlt wird und nach dieser Einstrahlung breitbandig oder selektiv bei einer Wellenlänge, insbesondere bei 270 nm eine Strahlungsintensität und/oder Wellenlänge der elektromagnetischen Strahlung im Innern des Bioreaktors von außerhalb gemessen wird.

Bei den hier offenbarten Verfahren können durch Mutagenese veränderte photo- oder mixotrophe Mikroorganismen, insbesondere auch Mikroalgen, Hefen und Bakterien verwendet werden.

Nachfolgend werden einige spezielle, nicht abschließend zu verstehende Ausführungsbeispiele der Erfindung, insbesondere Glass-To-Polymer-Seals (GTPS), Bezug nehmend auf die beigefügten Zeichnungen erläutert. Es zeigen:
Fig. 1: eine Schnittansicht eines Verbundelements gemäß einer ersten Ausführungsform, welche nicht durch die beanspruchte Erfindung abgedeckt ist,
Fig. 2: eine Schnittansicht eines Verbundelements gemäß einer zweiten Ausführungsform,
Fig. 3: eine Schnittansicht eines Verbundelements gemäß einer dritten Ausführungsform,
Fig. 4: eine Schnittansicht eines Verbundelements gemäß einer vierten Ausführungsform,
Fig. 5: eine Schnittansicht eines Verbundelements gemäß einer fünften Ausführungsform, welche nicht durch die beanspruchte Erfindung abgedeckt ist,
Fig. 6 und 7: Schnittansichten von Verbundelementen gemäß einer siebten Ausführungsform,
Fig. 8 und 9: eine Schnittansicht und eine Detailansicht eines Verbundelements gemäß einer sechsten Ausführungsform,
Fig. 10 und 11: eine Schnittansicht und eine Detailansicht eines Verbundelements gemäß einer achten Ausführungsform,
Fig. 12 und 13: eine Schnittansicht und eine Detailansicht eines Verbundelements gemäß einer neunten Ausführungsform,
Fig. 14 und 15: eine perspektivische Ansicht und eine Aufsicht einer Oberfläche eines Verbindungsteils mit Mikrostruktur,
Fig. 16 und 17: perspektivische Ansichten eines an einem Bioreaktor montierten Verbundelements gemäß der ersten Ausführungsform,
Fig. 18: eine Schnittansicht eines an einem Bioreaktor montierten Verbundelements gemäß der ersten Ausführungsform,
Fig. 19: eine perspektivische Detailansicht eines Verbundelements gemäß der ersten Ausführungsform,
Fig. 20: eine Seitenansicht eines Verbundelements gemäß einer zehnten Ausführungsform,
Fig. 21: eine Schnittansicht (durch die Ebene A-A in Fig. 22) des Verbundelements gemäß der zehnten Ausführungsform,
Fig. 22: eine Schnittansicht (durch die Ebene B-B aus Fig. 20) des Verbundelements gemäß der zehnten Ausführungsform,
Fig. 23, 24: perspektivische Ansichten des Verbundelements gemäß der zehnten Ausführungsform.

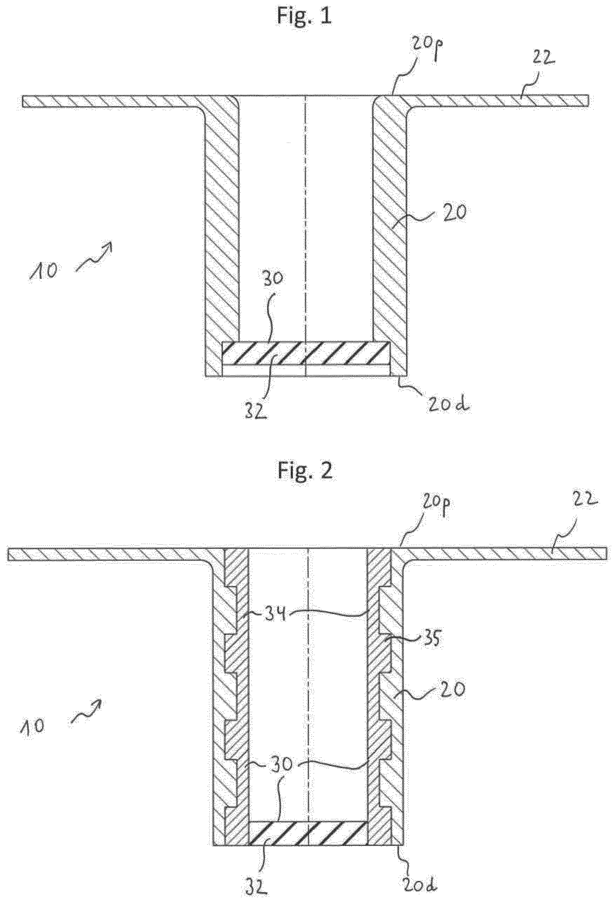

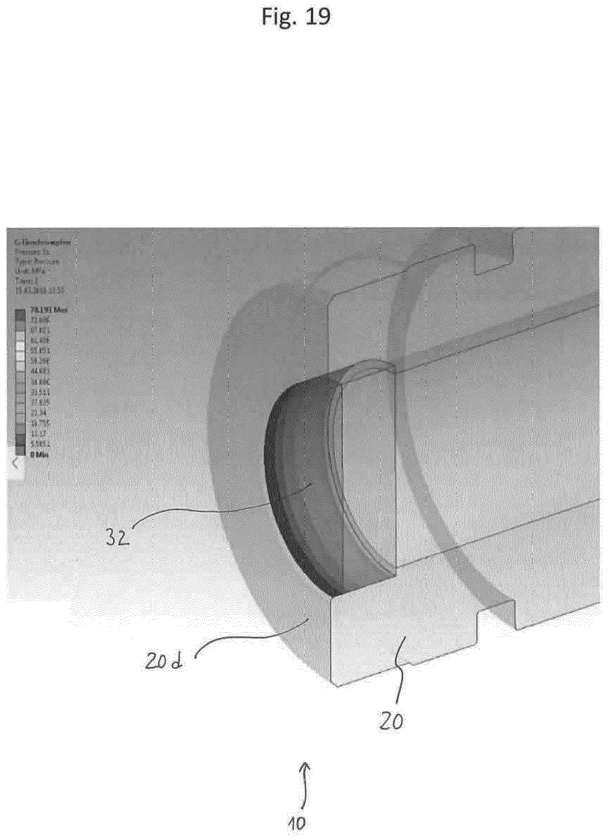

Das in Fig. 1 und Fig. 19 gezeigte Verbundelement (10), welches nicht durch die beanspruchte Erfindung abgedeckt ist, umfasst eine Außenfassung (20) aus einem Polymer, z.B. Polyetheretherketon (PEEK), und eine Innenkomponente (30) ausgebildet als einstückiges transparentes Bauteil (32) aus transparenten Material, z.B. Glas.

Bei dieser Ausführungsform handelt es sich um eine Druckeinpolymerisierung, welche z.B. wie folgt hergestellt werden kann: Das transparente Bauteil (32), insbesondere Glas, wird unter Druckspannung in die Polymer-Außenfassung (20) eingefügt.

Hierfür wird die als Polymerformteil ausgebildete Außenfassung (20) erwärmt (z.B. PEEK auf 200 °C), das in diesem Beispiel als Scheibe ausgebildete transparente Bauteil (32), mit einem größeren Durchmesser aus der Innendurchmesser des Ports eingesetzt, und abgekühlt. Daraus resultiert eine autoklavierbare und steril dichte Druckeinpolymerisierung.

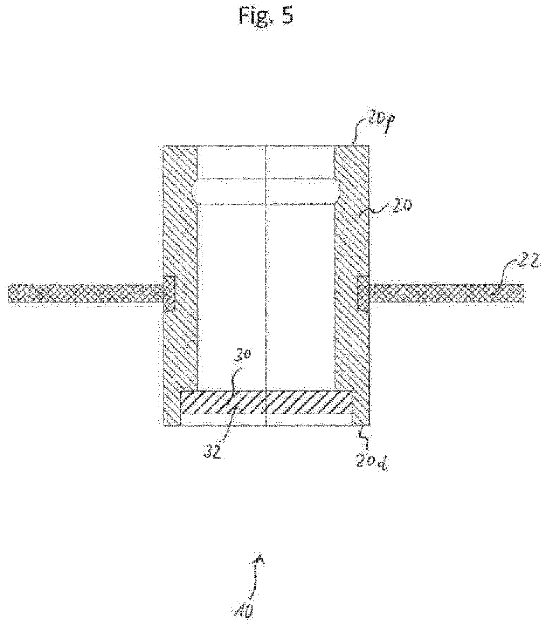

Die Polymer-Außenfassung (20) weist einen Flansch (22) auf, wobei der Flansch (22) an dem ersten (proximalen) Rohrende (20p) angeordnet ist. Ein Flansch (22) kann jedoch auch an anderer Stelle vorgesehen sein, insbesondere zwischen dem ersten Rohrende (20p) und dem zweiten Rohrende (20d), wie dargestellt in Fig. 5.

Das in Fig. 2 gezeigte Verbundelement (10) umfasst eine Außenfassung (20) aus einem Polymer, z.B. Polyethylen (PE), und eine zweiteilige Innenkomponente (30) mit einem scheibenförmigen transparenten Bauteil (32), z.B. aus Glas, und einem rohrförmigen Verbindungsteil (34), etwa aus Metall, z.B. 1.4404. In diesem Beispiel umschließt das Verbindungsteil (34) das transparente Bauteil (32).

Bei dieser Ausführungsform handelt es sich um einen Glas/Metal/Polymer-Verbund, der z.B. wie folgt herstellbar ist: Die Druckeinglasung des transparenten Bauteils (32) erfolgt steril dicht und/oder hermetisch dicht in dem Verbindungsteil (34), das in diesem Beispiel als profilierter Metallhohlzylinder ausgebildet ist. Das Verbindungsteil (34) bzw. die so entstandene Innenkomponente (30) wird mit einem Polymer, z.B. Polyethylen (PE), Polypropylen (PP), Polyamid (PA), Polyethylenterephthalat (PET), Ethylen-Vinyllalkohol (EVOH), Polyeterhetherketon (PEEK), Polyaryletherketon (PAEK), Polysulfon (PSU), Polyphenylensulfid (PPS),oder als Multimaterial Moulding mit mehreren Polymeren, auf Bauteilsolldimension steril dicht ummantelt, inklusive eines Flanschs (22). Die Außenfassung (20) kann demnach eines oder mehrere der vorstehend genannten Materialien umfassen oder daraus bestehen bzw. daraus hergestellt oder herstellbar sein.

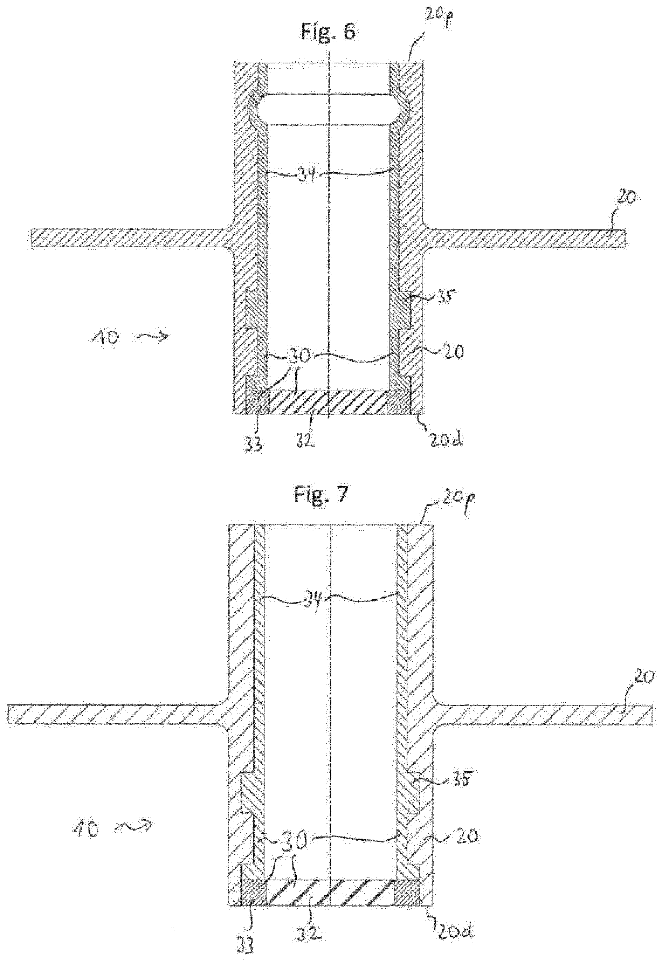

Die Polymer-Außenfassung (20) weist einen Flansch (22) am ersten Rohrende (20p) auf. Alternativ kann ein Flansch (22) jedoch auch an anderer Stelle vorgesehen sein, insbesondere zwischen dem ersten Rohrende (20p) und dem zweiten Rohrende (20d), wie dargestellt in Fig. 6 und Fig. 7.

Die in Fig. 6 und Fig. 7 gezeigten Verbundelemente weisen außerdem eine mehrteilige (hier: dreiteilige) Innenkomponente (30) auf, wobei das Verbindungsteil (34) sowohl mit der Außenfassung (20) unmittelbar verbunden ist als auch mit dem transparenten Bauteil (32) mittelbar verbunden ist. Das Verbindungsteil (34) ist stirnseitig mit einer das transparente Bauteil (32) umgebenden Fassung (33) verbunden, derart, dass das transparente Bauteil (32) steril dicht, bevorzugt hermetisch dicht, in der Fassung (33) aufgenommen ist, die Fassung (33) wiederum steril dicht, bevorzugt hermetisch dicht, mit dem Verbindungsteil (34) verbunden ist und das Verbindungsteil (34) wiederum steril dicht, bevorzugt hermetisch dicht, in der Außenfassung (20) aufgenommen ist.

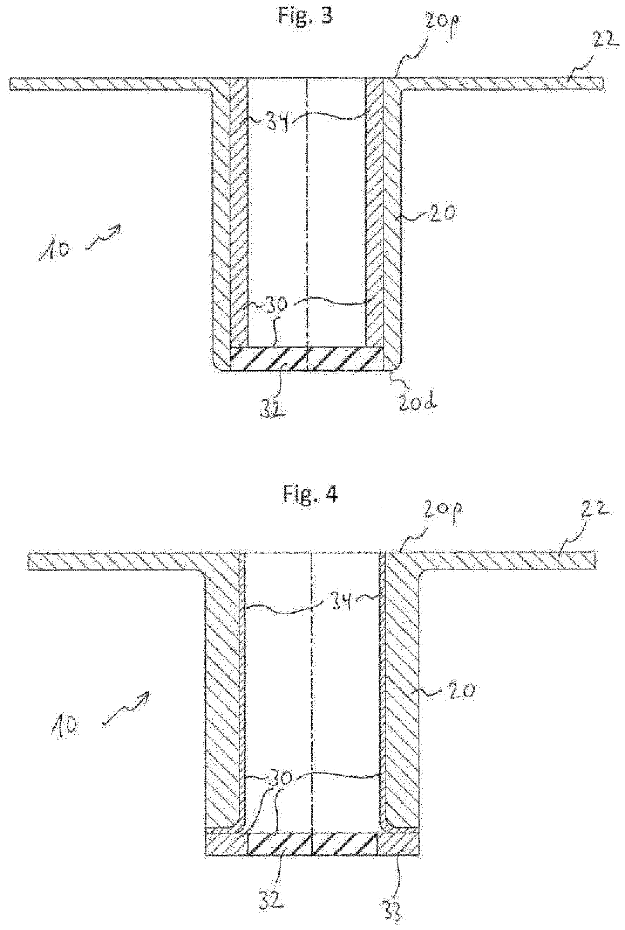

Das in Fig. 3 gezeigte Verbundelement (10) umfasst wiederum eine Polymer-Außenfassung (20) und eine zweiteilige Innenkomponente (30) mit transparentem Bauteil (32) und rohrförmigem Verbindungsteil (34), wobei in diesem Beispiel das Verbindungsteil (34) als Hohlzylinder aus transparentem Material, insb. Glas, ausgebildet ist. Ferner grenzt das Verbindungsteil (34) in diesem Fall stirnseitig an das transparente Bauteil (32) an.

Bei dieser Ausführungsform handelt es sich um einen Glas/Glas/Polymer-Verbund, welcher beispielsweise wie folgt hergestellt werden kann: Das insbesondere als Glaszylinder ausgebildete Verbindungsteil (34) wird stirnseitig geschliffen und poliert, außerdem wird das z.B. als Scheibe ausgebildete transparente Bauteil (32) geschliffen und poliert, und zwar werden diese Bauteile so geschliffen und poliert, dass kohärente Flächen entstehen.

Danach werden die beiden Teile so verbunden, dass sie über zwischenmolekulare Kräfte aneinanderhaften (d.h. die Scheibe wird an den Glaszylinder angesprengt). Nachfolgend können die beiden Teile fest verbunden werden, beispielsweise durch Laserschweißen, um so eine dauerhaft steril dichte Verbindung herzustellen. Die so entstandene Innenkomponente (30) wird wiederum mit einem Polymer, z.B. Polyethylen (PE), auf Bauteilsolldimension steril dicht ummantelt, inklusive eines Flanschs (22).

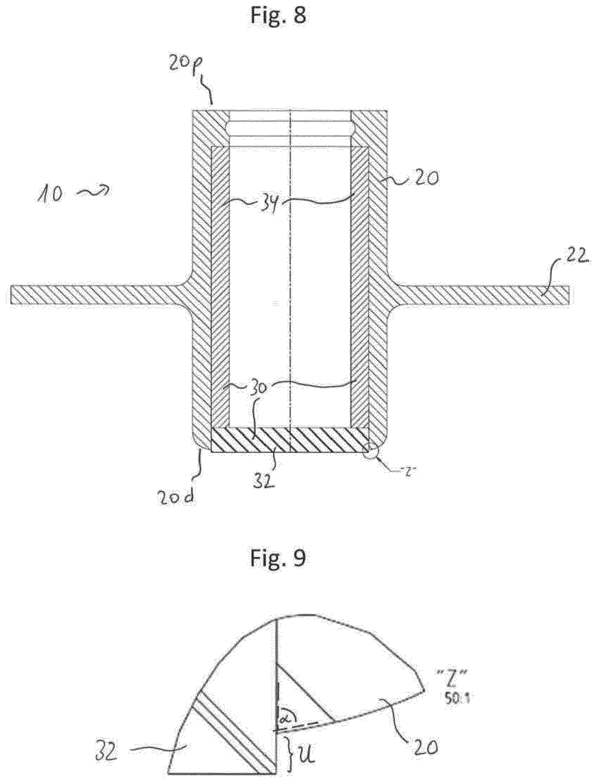

Durch die Ummantelung entsteht die Polymer-Außenfassung (20), welche in diesem Beispiel einen Flansch (22) an dem ersten (proximalen) Rohrende (20p) aufweist. Ein Flansch (22) kann jedoch auch an anderer Stelle gebildet werden, insbesondere zwischen dem ersten Rohrende (20p) und dem zweiten Rohrende (20d), wie dargestellt in Fig. 8.

Fig. 9 zeigt darüber hinaus eine Detailansicht des in Fig. 8 gezeigten Verbindungselements (10). Das transparente Bauteil (32) ragt hierbei aus der Außenfassung (20) heraus, so dass ein Überstand U gebildet wird. Da der Benetzungswinkel des Materials der Außenfassung auf dem Material des transparenten Bauteils (20) kleiner als 90° ist, entsteht zwischen er Außenfassung (20) und dem transparenten Bauteil (32) entsprechend ein Winkel α von kleiner als 90°.

Das in Fig. 4 gezeigte Verbundelement (10) umfasst eine Polymer-Außenfassung (20) und eine mehrteilige Innenkomponente (30) mit transparentem Bauteil (32), einer das transparente Bauteil (32) umgebende Fassung (33) und rohrförmigem Verbindungsteil (34). Das Verbindungsteil (34) ist stirnseitig mit der das transparente Bauteil (32) umgebenden Fassung (33) verbunden, derart, dass das transparente Bauteil (32) steril dicht, bevorzugt hermetisch dicht, in der Fassung (33) aufgenommen ist, die Fassung (33) wiederum steril dicht, bevorzugt hermetisch dicht, mit dem Verbindungsteil (34) verbunden ist und das Verbindungsteil (34) wiederum steril dicht, bevorzugt hermetisch dicht, in der Außenfassung (20) aufgenommen ist.

In diesem Beispiel ragt die Innenkomponente (30) zumindest teilweise aus der Außenfassung (20) heraus, insbesondere mit dem transparenten Bauteil (32) und der Fassung (33). Es kann aber alternativ auch vorgesehen sein, dass die Außenfassung (20) die Innenkomponente (30) der Länge nach vollständig umschließt.

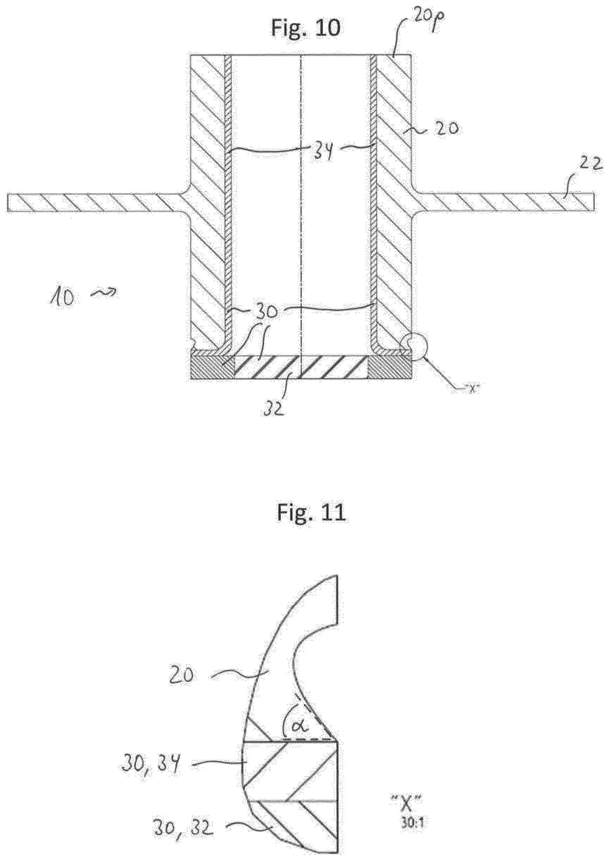

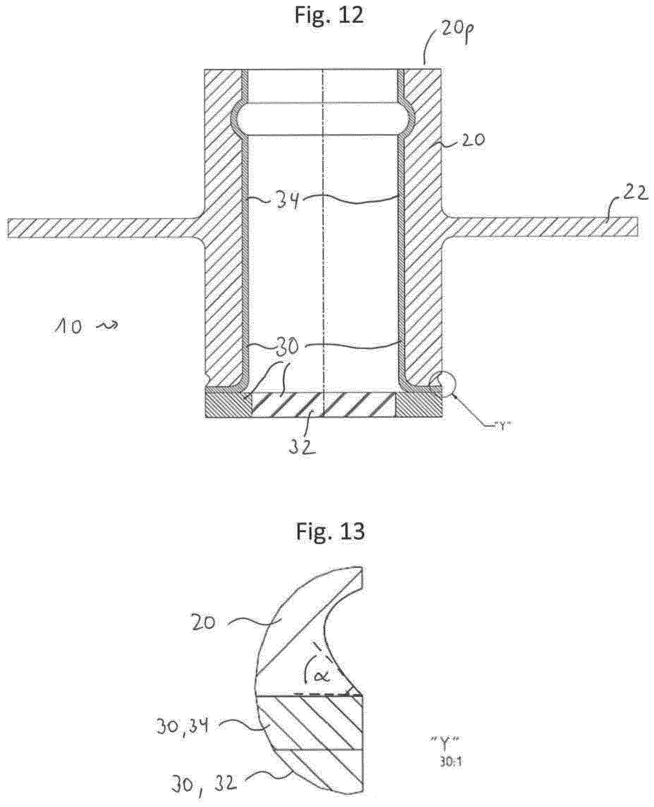

Die Außenfassung (20) weist in diesem Beispiel wiederum einen Flansch (22) an dem ersten Rohrende (20p) auf. Ein Flansch (22) kann jedoch auch an anderer Stelle vorgesehen sein, insbesondere zwischen dem ersten Rohrende (20p) und dem zweiten Rohrende (20d), wie dargestellt in Fig. 10 und Fig. 12.

Fig. 11 und Fig. 13 zeigt darüber hinaus Detailansichten der in Fig. 10 bzw. Fig. 12 gezeigten Verbindungselemente (10). Zwischen der Außenfassung (20) und der Innenkomponente (30) ist ein Winkel α von kleiner als 90° vorgesehen. Dazu ist in der Außenfassung (20) eine bogenförmige Ausnehmung (23) eingebracht. Diese Ausnehmung (23) befindet sich im Polymermaterial am Materialübergang zur Innenkomponente (30).

Fig. 14 und 15 zeigen eine Oberfläche eines Verbindungsteils in welchem eine Mikrostruktur in Form zahlreicher Vorsprünge (36) eingebracht ist. Durch eine erste Schar von Rillen (37'), welche von einer zweiten Schar von Rillen (37") gekreuzt wird, entsteht die Mehrzahl von Vorsprüngen (36) zwischen den Rillen.

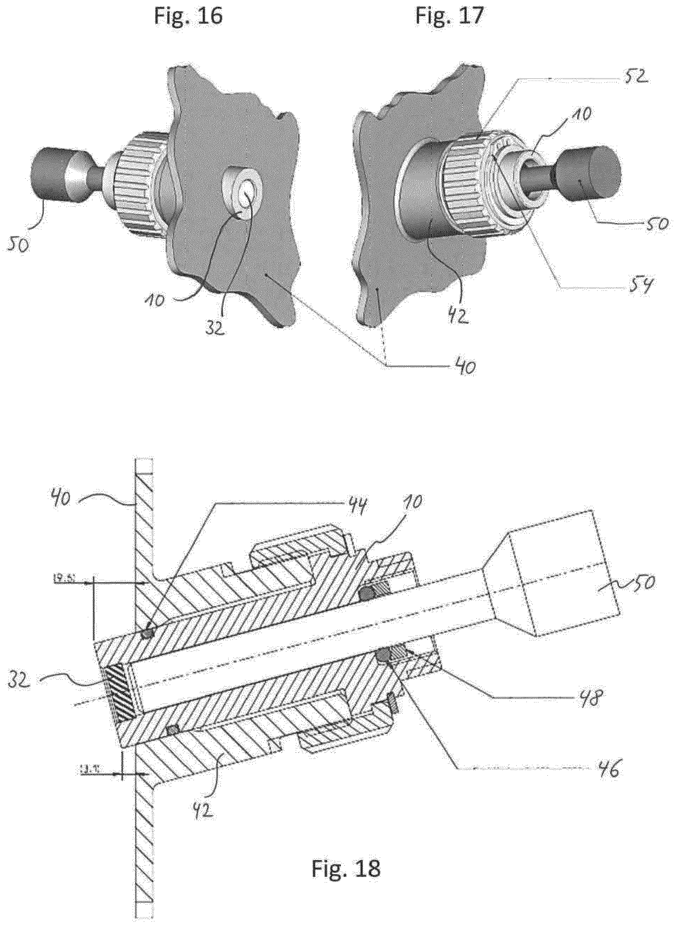

Die Fig. 16, 17 und 18 zeigen eine Bioreaktorwand (40) als Teil eines Bioreaktors mit einer darin befindlichen Durchführung (42), auch Port (42) genannt, die eine Öffnung in das Innere des Bioreaktors bildet, wobei sich ein Verbundelement (10) zumindest teilweise in der durch die Durchführung (42) gebildeten Öffnung erstreckt. Das Verbundelement (10) ist in diesem Beispiel ausgebildet gemäß dem in Fig. 1 gezeigten Verbundelement (10). Selbstverständlich kann sich aber jedes vorstehend beschriebene Verbundelement (10) durch die hier dargestellte Durchführung (42) erstrecken.

Im Inneren des Verbundelements (10) ist eine Messsonde (50) installiert, die es ermöglicht, Messungen im Inneren des Bioreaktors durchzuführen, wobei die Messung durch das transparente Bauteil (32) des Verbundelements (10) erfolgt. Mittels eines Dichtelements (44), beispielsweise eines O-Rings, ist das Verbundelement (10) vorzugsweise fluiddicht und besonders bevorzugt steril dicht und/oder hermetisch dicht gegenüber der Durchführung (42) der Bioreaktorwand (40), insbesondere form- und reibschlüssig, gehalten. Die Messsonde (50) geht wiederum mittels eines Reibelements (46), vorzugsweise einem O-Ring, eine reibschlüssige Verbindung zu dem Verbundelement (10) ein, wobei das Reibelement (46) in einer zylindrischen Ausnehmung des Verbundelements durch ein, insbesondere im Wesentlichen ringförmiges, Druckelement (Druckring) (48) gehalten ist, welches in axialer Richtung des Verbundelements (10) eine definiert einstellbare Kraft auf das Reibelement (46) ausübt.

Mittels einer insbesondere als Hutmutter ausgebildeten Überwurfmutter (52) ist das Verbundelement (10) lösbar, jedoch ortsfest an der Durchführung (42) der Bioreaktorwand (40) gehalten. Mittels eines Sicherungsrings bzw. Sprengrings (54) ist die Überwurfmutter (52) drehbar jedoch axial nur mit geringem Spiel unverlierbar an dem Verbundelement (10) gehalten.

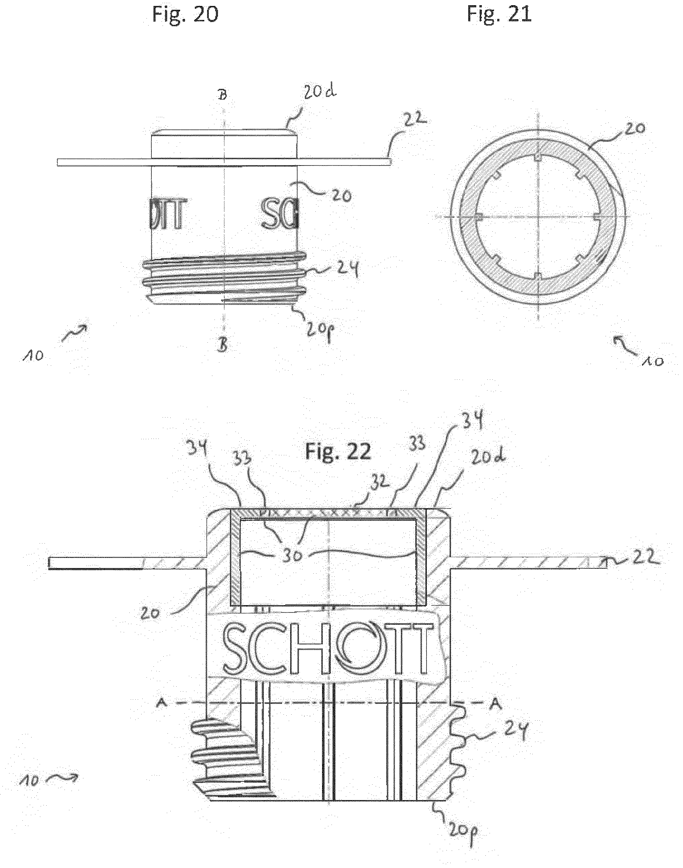

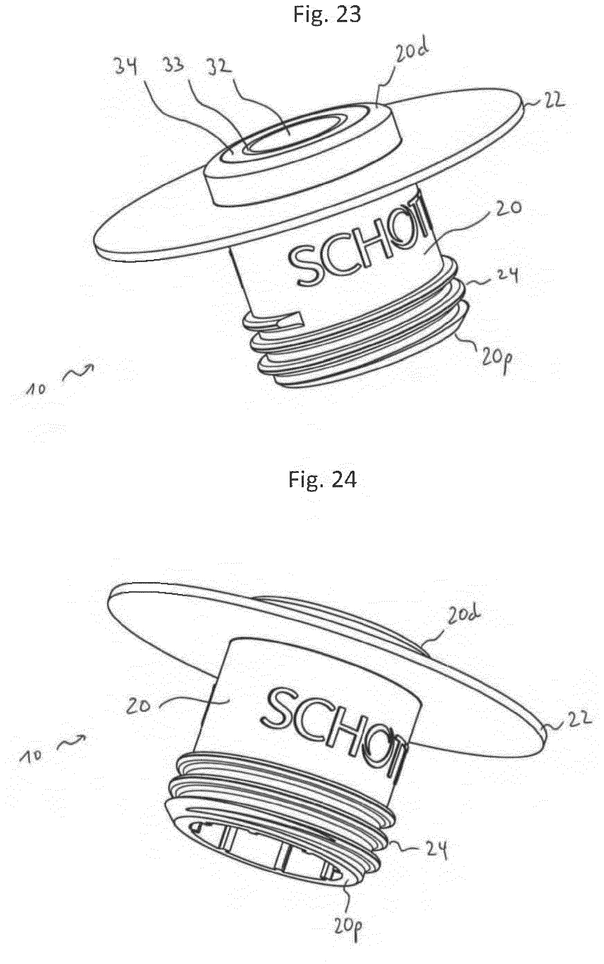

Die Fig. 20 bis 24 zeigen verschiedene Ansichten eines biokompatiblen Verbundelements (10) mit einer Außenfassung (20) bestehend aus oder umfassend ein Polymermaterial, z.B. PE, sowie einer mehrteiligen Innenkomponente (30).

Die Außenfassung (20) ist rohrförmig ausgebildet und weist zwischen den beiden Rohrenden (20p, 20d) einen Flansch (22) auf. Der Flansch befindet sich in diesem Beispiel näher an dem zweiten Rohrende (20d) als an dem ersten Rohrende (20p), wobei dies generell für jede Ausführungsform vorgesehen sein kann. Am ersten Rohrende (20p) weist die Außenfassung (20) ein Gewinde (24) auf, wobei auch dies generell für jede Ausführungsform vorgesehen sein kann.

Die Innenkomponente (30), welche in diesem Beispiel dreiteilig ausgebildet ist, umfasst ein rohrförmiges Verbindungsteil (34) bestehend aus oder umfassend ein Metall, insbesondere einen nichtrostenden austenitisch-ferritischen Duplex-Stahl, z.B. 1.4462. Am zweiten Rohrende (20d) ist ein transparentes Bauteil (32), z.B. mit oder aus Saphir, in dem Verbindungsteil (34) aufgenommen. Das transparente Bauteil (32) ist dabei, insbesondere unmittelbar, mit einer umgebenden Fassung (33) verbunden, welche z.B. mit oder aus Glas gefertigt ist und welche ihrerseits, insbesondere unmittelbar, mit dem Verbindungsteil (34) verbunden ist. Hierdurch ist das transparente Bauteil (32) steril dicht, bevorzugt hermetisch dicht, in der Fassung (33) aufgenommen, die Fassung (33) wiederum steril dicht, bevorzugt hermetisch dicht, mit dem Verbindungsteil (34) verbunden und das Verbindungsteil (34) wiederum steril dicht, bevorzugt hermetisch dicht, in der Außenfassung (20) aufgenommen, derart, dass das transparente Bauteil (32) steril dicht, bevorzugt hermetisch dicht mit dem Verbindungsteil (34) verbunden ist und das Verbindungsteil (34) wiederum steril dicht, bevorzugt hermetisch dicht, in der Außenfassung (20) aufgenommen ist. Wie beschrieben kann die Fassung (33) z.B. Glas umfassen oder daraus bestehen, wobei z.B. zunächst ein Pressling mit oder aus Glaspulver vorgesehen sein kann. Das biokompatible Verbundelement (10) kann dann etwa herstellbar oder hergestellt sein durch Erhitzen, beispielsweise in einem Durchlaufofen, so dass ein Glas-Metall-Verbund entsteht.

## Patentansprüche

1. Biokompatibles Verbundelement (10), insbesondere zur Verwendung als Anschlussstück für Bioreaktoren, vorzugsweise Einweg-Bioreaktoren, um ein Fenster in den Bioreaktor zu bilden, umfassend:
- eine Außenfassung (20) mit oder aus einem Polymermaterial, insbesondere zum Anschließen an eine Wandung des Bioreaktors, insbesondere zum untrennbaren Anschließen an die Wandung des Einweg-Bioreaktors, und
- eine Innenkomponente (30) welche zumindest zweiteilig ausgebildet ist, nämlich mit einem transparenten Bauteil (32) und einem Verbindungsteil (34), wobei das Verbindungsteil (34) sowohl mit dem transparenten Bauteil (32) unmittelbar oder mittelbar verbunden ist als auch mit der Außenfassung (20) verbunden ist, wobei die Innenkomponente steril dicht in der Außenfassung aufgenommen ist und ein Fenster bildet, insbesondere um von außerhalb des Bioreaktors eine spektrale Prozesskontrolle zu ermöglichen.

2. Biokompatibles Verbundelement (10) nach Anspruch 1,
- wobei die Außenfassung (20) ringförmig ausgebildet ist, derart, dass die Außenfassung die Innenkomponente im Querschnitt umschließt und/oder
- wobei die Außenfassung (20) rohrförmig mit einem ersten und einem zweiten Rohrende (20p, 20d) ausgebildet ist, insbesondere derart, dass die Außenfassung (20) mit dem ersten Rohrende (20p) an die Wandung des Bioreaktors anschließbar ist, und/oder
- wobei die Außenfassung einen Flansch (22) aufweist, insbesondere zum Anschließen an die Wandung des Bioreaktors, insbesondere zum untrennbaren Anschließen an die Wandung des Einweg-Bioreaktors, und wobei der Flansch (22) bevorzugt zwischen dem ersten und dem zweiten Rohrende oder an dem ersten Rohrende (20p) angeordnet ist und/oder
- wobei die Außenfassung ein Gewinde (24) aufweist, welches insbesondere als Außengewinde ausgebildet ist, wobei das Gewinde (24) bevorzugt an dem ersten Rohrende (20p) angeordnet ist.

3. Biokompatibles Verbundelement (10) nach Anspruch 1 oder 2,
- wobei das transparente Bauteil (32) der Innenkomponente (30) ausgebildet ist als scheibenförmiges transparentes Bauteil (32).

4. Biokompatibles Verbundelement (10) nach Anspruch 1 oder 2,
- wobei das Verbindungsteil (34) der Innenkomponente (30) ausgebildet ist als rohrförmiges Verbindungsteil (34).

5. Biokompatibles Verbundelement nach einem der Ansprüche 1 bis 4,
- wobei das Verbindungsteil (34) das transparente Bauteil (32) im Querschnitt umschließt, derart, dass das transparente Bauteil (32) steril dicht in dem Verbindungsteil (34) aufgenommen ist und/oder mit dem Verbindungsteil (34) verbunden ist und das Verbindungsteil (34) wiederum steril dicht in der Außenfassung (20) aufgenommen ist, insbesondere untrennbar aufgenommen ist, und/oder
- wobei das Verbindungsteil (34) eine das transparente Bauteil (32) umgebende Fassung (33) im Querschnitt umschließt, derart, dass das transparente Bauteil (32) steril dicht in der Fassung (33) aufgenommen ist, die Fassung (33) wiederum steril dicht in dem Verbindungsteil (34) aufgenommen ist und das Verbindungsteil (34) wiederum steril dicht in der Außenfassung (20) aufgenommen ist.

6. Biokompatibles Verbundelement (10) nach einem der Ansprüche 1 bis 4,
- wobei das Verbindungsteil (34) stirnseitig an das transparente Bauteil (32) angrenzt und/oder stirnseitig mit dem transparenten Bauteil (32) verbunden ist, derart, dass sowohl das transparente Bauteil (32) als auch das Verbindungsteil (34) jeweils steril dicht in der Außenfassung (20) aufgenommen sind, insbesondere untrennbar aufgenommen sind.

7. Biokompatibles Verbundelement (10) nach einem der Ansprüche 1 bis 4,
- wobei das Verbindungsteil (34) stirnseitig mit dem transparenten Bauteil (32) und/oder einer das transparente Bauteil (32) umgebenden Fassung (33) verbunden ist, derart, dass das transparente Bauteil (32) steril dicht in der Fassung (33) aufgenommen ist, die Fassung (33) wiederum steril dicht mit dem Verbindungsteil (34) verbunden ist und das Verbindungsteil (34) wiederum steril dicht in der Außenfassung (20) aufgenommen ist und/oder derart, dass das transparente Bauteil (32) steril dicht mit dem Verbindungsteil (34) verbunden ist und das Verbindungsteil (34) wiederum steril dicht in der Außenfassung (20) aufgenommen ist und/oder
- wobei die Innenkomponente (30) zumindest teilweise aus der Außenfassung (20) herausragt, insbesondere mit dem transparenten Bauteil (32) und/oder der Fassung (33) aus der Außenfassung (20) herausragt.

8. Biokompatibles Verbundelement (10) nach einem der Ansprüche 1 bis 7,
- wobei die Innenkomponente (30) unter Druckspannung stehend in der Außenfassung (20) aufgenommen ist, insbesondere untrennbar aufgenommen ist.

9. Biokompatibles Verbundelement (10) nach einem der Ansprüche 1 bis 7,
- wobei die Innenkomponente (30) spannungsneutral in der Außenfassung (20) aufgenommen ist, insbesondere untrennbar aufgenommen ist.

10. Biokompatibles Verbundelement (10) nach Anspruch 8 oder 9,
- wobei das transparente Bauteil (32) unter Druckspannung stehend in dem Verbindungsteil (34) aufgenommen ist, insbesondere untrennbar aufgenommen ist.

11. Biokompatibles Verbundelement (10) nach Anspruch 8 oder 9,
- wobei das transparente Bauteil (32) spannungsneutral mit dem Verbindungsteil (34) verbunden ist, insbesondere untrennbar verbunden ist.

12. Verfahren zur Herstellung eines biokompatiblen Verbundelements (10) mit einer Außenfassung (20) mit oder aus einem Polymermaterial und einer Innenkomponente welche zumindest zweiteilig ausgebildet ist, nämlich mit einem transparenten Bauteil (32) und einem Verbindungsteil (34), wobei das Verbindungsteil (34) sowohl mit dem transparenten Bauteil (32) unmittelbar oder mittelbar verbunden ist als auch mit der Außenfassung (20) verbunden ist, wobei die Innenkomponente steril dicht in der Außenfassung aufgenommen ist und ein Fenster bildet, umfassend:
- Bereitstellen der Außenfassung (20) mit oder aus einem Polymermaterial und der Innenkomponente (30),
- Herbeiführen eines relativen Ausdehnens der Außenfassung (20) gegenüber der Innenkomponente (30), insbesondere durch Erwärmen der Außenfassung oder vorzugsweise durch gemeinsames Erwärmen der Außenfassung und der Innenkomponente,
- Einsetzen der Innenkomponente (30) in die gegenüber der Innenkomponente (30) ausgedehnten Außenfassung (20),
- Herbeiführen eines relativen Zusammenziehens der Außenfassung (20) gegenüber der Innenkomponente (30), insbesondere durch Abkühlen der Außenfassung oder vorzugsweise durch gemeinsames Abkühlen der Außenfassung und der Innenkomponente, so dass die Außenfassung (20) die Innenkomponente (30) steril dicht aufnimmt.

13. Verfahren zur Herstellung eines biokompatiblen Verbundelements (10) mit einer Außenfassung (20) mit oder aus einem Polymermaterial und einer Innenkomponente welche zumindest zweiteilig ausgebildet ist, nämlich mit einem transparenten Bauteil (32) und einem Verbindungsteil (34), wobei das Verbindungsteil (34) sowohl mit dem transparenten Bauteil (32) unmittelbar oder mittelbar verbunden ist als auch mit der Außenfassung (20) verbunden ist, wobei die Innenkomponente steril dicht in der Außenfassung aufgenommen ist und ein Fenster bildet, umfassend:
- Bereitstellen der Innenkomponente (30) und flüssigen Polymermaterials zur Bildung der Außenfassung, wobei das Polymermaterial mit dem oder einem Material der Innenkomponente (30) vorzugsweise einen Benetzungswinkel ausbildet, welcher geringer ist als 90°,
- Zuführen des flüssigen Polymermaterials von außen an die Innenkomponente (30), insbesondere an das Verbindungsteil (34) und/oder an das transparente Bauteil (32),
- Aushärten des flüssigen Polymermaterials, derart, dass eine die Innenkomponente (30) steril dicht aufnehmende Außenfassung (20) gebildet wird.

14. Verfahren nach Anspruch 13 zur Herstellung eines biokompatiblen Verbundelements (10), insbesondere nach Anspruch 10, umfassend:
- Bereitstellen eines transparenten Bauteils (32) und eines Verbindungsteils (34), insbesondere aus Metall,
- Herbeiführen eines relativen Ausdehnens des Verbindungsteils (34) gegenüber dem transparenten Bauteil (32), insbesondere durch Erwärmen des Verbindungsteils oder vorzugsweise durch gemeinsames Erwärmen des Verbindungsteils und des transparenten Bauteils,
- Einsetzen des transparenten Bauteils (32) in das gegenüber dem transparenten Bauteil (32) ausgedehnten Verbindungsteil (34),
- Herbeiführen eines relativen Zusammenziehens des Verbindungsteils (34) gegenüber dem transparenten Bauteil (32), insbesondere durch Abkühlen des Verbindungsteils oder vorzugsweise durch gemeinsames Abkühlen des Verbindungsteils und des transparenten Bauteils, so dass sich das Verbindungsteil (34) mit dem transparente Bauteil (32) zu der Innenkomponente (30) verbindet,
- Ausführen der Verfahrensschritte nach Anspruch 13.

15. Verfahren nach Anspruch 13 zur Herstellung eines biokompatiblen Verbundelements (10), insbesondere nach Anspruch 11, umfassend:
- Bereitstellen eines transparenten Bauteils (32) und eines Verbindungsteils (34), insbesondere aus demselben Material wie das transparente Bauteil (32),
- Heranführen und/oder Ansprengen des Verbindungsteils (34) stirnseitig an das transparente Bauteil (32) und vorzugsweise Laserverschweißen des Verbindungsteils (34) mit dem transparenten Bauteil (32), so dass sich das Verbindungsteil (34) mit dem transparente Bauteil (32) zu der Innenkomponente (30) verbindet,
- Ausführen der Verfahrensschritte nach Anspruch 13.

16. Verfahren nach einem der Ansprüche 12 bis 15 zur Herstellung eines biokompatiblen Verbundelements (10) umfassend:
- Sterilisieren, insbesondere Autoklavieren, des Verbundelements (10) umfassend die Außenfassung (20) und die darin steril dicht, bevorzugt hermetisch dicht aufgenommene Innenkomponente (30).

17. Bioreaktor, insbesondere Einweg-Bioreaktor, zur Kultivierung von Mikroorgansimen oder Zellen mit einem an eine Wandung des Bioreaktors als Anschlussstück angeschlossenen Verbundelement (10), insbesondere nach einem der Ansprüche 1 bis 11,
- wobei die Außenfassung (20) an der Wandung des Bioreaktors angeschlossen ist, insbesondere damit steril dicht verbunden ist und
- wobei die Innenkomponente ein Fenster in den Bioreaktor bildet, insbesondere um von außerhalb des Bioreaktors eine spektrale Prozesskontrolle zu ermöglichen.

18. Verfahren zur Vermehrung oder Kultivierung biologischen Materials in einem Bioreaktor nach Anspruch 17, insbesondere umfassend die Herstellung von Pharmazeutika, insbesondere von Biopharmazeutika, mit einem an eine Wandung des Bioreaktors als Anschlussstück angeschlossenen biokompatiblen Verbundelement (10) nach einem der Ansprüche 1 bis 11, wobei von außerhalb des Bioreaktors eine spektrale Prozesskontrolle erfolgt, wobei insbesondere eine physikalischen, chemischen oder biologischen Messgröße von außerhalb des Bioreaktors durch das gebildete Fenster erfasst wird.

## Claims

1. A biocompatible composite element (10), in particular for use as a fitting for bioreactors, preferably single-use bioreactors, to provide a window into the bioreactor, comprising:
- an outer frame (20) including or made of a polymer material, in particular for being connected to a wall of the bioreactor, in particular for being inseparably connected to the wall of the single-use bioreactor; and
- an inner component (30) consisting of at least two parts, namely comprising a transparent component (32) and a coupling part (34), wherein the coupling part (34) is directly or indirectly joined to the transparent component (32) and is also joined to the outer frame (20), wherein the inner component is retained in the outer frame in a sterile sealed manner and defines a window, in particular in order to enable spectral process control from outside the bioreactor.

2. The biocompatible composite element (10) according to claim 1,
- wherein the outer frame (20) has an annular shape such that the outer frame encloses the inner component in cross section; and/or
- wherein the outer frame (20) has a tubular shape with first and second tube ends (20p, 20d), in particular such that the first tube end (20p) of the outer frame (20) is connectable to the wall of the bioreactor; and/or
- wherein the outer frame has a flange (22), in particular for being connected to the wall of the bioreactor, in particular for being inseparably connected to the wall of the single-use bioreactor, and wherein the flange (22) is preferably provided between the first and the second tube ends or at the first tube end (20p); and/or
- wherein the outer frame has a thread (24) which is in particular in the form of an external thread, wherein the thread (24) is preferably provided at the first tube end (20p).

3. The biocompatible composite element (10) according to claim 1 or 2,
- wherein the transparent component (32) of the inner component (30) is in the form of a sheet-like transparent component (32).

4. The biocompatible composite element (10) according to claim 1 or 2,
- wherein the coupling part (34) of the inner component (30) is in the form of a tubular coupling part (34).

5. The biocompatible composite element according to any one of claims 1 to 4,
- wherein the coupling part (34) encloses the transparent component (32) in cross section such that the transparent component (32) is retained in the coupling part (34) in a sterile sealed manner and/or is joined to the coupling part (34) and the coupling part (34) in turn is retained, in particular inseparably retained in the outer frame (20) in a sterile sealed manner; and/or
- wherein the coupling part (34) encloses a frame (33) surrounding the transparent component (32) in cross section, such that the transparent component (32) is retained in the frame (33) in a sterile sealed manner, the frame (33), in turn, is retained in the coupling part (34) in a sterile sealed manner, and the coupling part (34), in turn, is retained in the outer frame (20) in a sterile sealed manner.

6. The biocompatible composite element (10) according to any one of claims 1 to 4,
- wherein the coupling part (34) engages on the transparent component (32) with an end face thereof and/or is joined to the transparent component (32) at an end face thereof such that both the transparent component (32) and the coupling part (34) are retained, in particular inseparably retained in the outer frame (20) in a sterile sealed manner.

7. The biocompatible composite element (10) according to any one of claims 1 to 4,
- wherein the front end of the coupling part (34) is joined to the transparent component (32) and/or to a frame (33) surrounding the transparent component (32) in such a way that the transparent component (32) is retained in the frame (33) in a sterile sealed manner, wherein the frame (33), in turn, is joined to the coupling part (34) in a sterile sealed manner and the coupling part (34), in turn, is retained in the outer frame (20) in a sterile sealed manner, and/or in such a way that the transparent component (32) is joined to the coupling part (34) in a sterile sealed manner and the coupling part (34), in turn, is retained in the outer frame (20) in a sterile sealed manner; and/or
- wherein the inner component (30) protrudes at least partially from the outer frame (20), in particular with the transparent component (32) and/or the frame (33) protruding from the outer frame (20).

8. The biocompatible composite element (10) according to any one of claims 1 to 7,
- wherein the inner component (30) is retained, in particular inseparably retained in the outer frame (20) so as to be under compressive stress.

9. The biocompatible composite element (10) according to any one of claims 1 to 7,
- wherein the inner component (30) is retained, in particular inseparably retained in the outer frame (20) in a stress-neutral manner.

10. The biocompatible composite element (10) according to claim 8 or 9,
- wherein the transparent component (32) is retained, in particular inseparably retained in the coupling part (34) so as to be under compressive stress.

11. The biocompatible composite element (10) according to claim 8,
- wherein the transparent component (32) is retained, in particular inseparably retained in the coupling part (34) in a stress-neutral manner.

12. A method for producing a biocompatible composite element (10) comprising an outer frame (20) that includes or is made of a polymer material, and an inner component consisting of at least two parts, namely comprising a transparent component (32) and a coupling part (34), wherein the coupling part (34) is directly or indirectly joined to the transparent component (32) and is also joined to the outer frame (20), wherein the inner component is retained in the outer frame in a sterile sealed manner and defines a window, comprising:
- providing the outer frame (20) including or made of a polymer material, and the inner component (30);
- causing the outer frame (20) to expand relative to the inner component (30), in particular by heating the outer frame or preferably by jointly heating the outer frame and the inner component;
- placing the inner component (30) in the outer frame (20) that has been expanded relative to the inner component (30);
- causing the outer frame (20) to contract relative to the inner component (30), in particular by cooling the outer frame or preferably by jointly cooling the outer frame and the inner component, such that the outer frame (20) retains the inner component (30) in a sterile sealed manner.

13. A method for producing a biocompatible composite element (10) comprising an outer frame (20) that includes or is made of a polymer material, and an inner component consisting of at least two parts, namely comprising a transparent component (32) and a coupling part (34), wherein the coupling part (34) is directly or indirectly joined to the transparent component (32) and is also joined to the outer frame (20), wherein the inner component is retained in the outer frame in a sterile sealed manner and defines a window, comprising:
- providing the inner component (30) and liquid polymer material to form the outer frame, wherein the polymer material preferably forms a wetting angle of less than 90° with the one or a material of the inner component (30);
- feeding the liquid polymer material to the inner component (30) from outside, in particular to the coupling part (34) and/or to the transparent component (32);
- curing the liquid polymer material such that an outer frame (20) is formed retaining the inner component (30) in a sterile sealed manner.

14. The method according to claim 13 for producing a biocompatible composite element (10) in particular according to claim 10, comprising:
- providing a transparent component (32) and a coupling part (34), in particular made of metal;
- causing the coupling part (34) to expand relative to the transparent component (32), in particular by heating the coupling part or preferably by jointly heating the coupling part and the transparent component;
- placing the transparent component (32) in the coupling part (34) that has been expanded relative to the transparent component (32);
- causing the coupling part (34) to contract relative to the transparent component (32), in particular by cooling the coupling part or preferably by jointly cooling the coupling part and the transparent component, such that the coupling part (34) is bonded to the transparent component (32) to form the inner component (30);
- performing the method steps according to claim 13.

15. The method according to claim 13 for producing a biocompatible composite element (10), in particular according to claim 11, comprising:
- providing a transparent component (32) and a coupling part (34), in particular made of the same material as the transparent component (32);
- bringing an end face of the coupling part (34) close to the transparent component (32) and/or optical contact bonding it thereto, and preferably laser welding the coupling part (34) to the transparent component (32) such that the coupling part (34) is bonded to the transparent component (32) to form the inner component (30);
- performing the method steps according to claim 13.

16. The method according to any one of claims 12 to 15 for producing a biocompatible composite element (10), comprising:
- sterilizing, in particular autoclaving, the composite element (10) comprising the outer frame (20) and the inner component (30) that is retained therein in a sterile sealed manner.

17. A bioreactor, in particular a single-use bioreactor, for cultivation of microorganisms or cells, comprising a fitting in the form of a composite element (10) according to any one of claims 1 to 11 connected to a wall of the bioreactor;
- wherein the outer frame (20) is connected to the wall of the bioreactor, in particular connected thereto in a sterile sealed manner; and
- wherein the inner component provides a window into the bioreactor, in particular to allow for spectral process control from outside the bioreactor.

18. A method for propagation or cultivation of biological material in a bioreactor according to claim 17, in particular comprising the production of pharmaceuticals, in particular biopharmaceuticals, which comprises a fitting in the form of a biocompatible composite element (10) according to any one of claims 1 to 11 connected to a wall of the bioreactor, wherein spectral process control is conducted from outside the bioreactor, wherein in particular a physical, chemical or biological parameter is detected from outside the bioreactor through the provided window.

## Revendications

1. Élément composite biocompatible (10), destiné notamment à une utilisation en tant que pièce de raccordement pour des bioréacteurs, de préférence des bioréacteurs à usage unique, afin de réaliser une fenêtre dans le bioréacteur, comprenant :
- un support extérieur (20) comportant un matériau polymère ou constitué d'un tel matériau, destiné notamment au raccordement à une paroi du bioréacteur, en particulier au raccordement non séparable à la paroi du bioréacteur à usage unique, et
- un composant interne (30) qui est réalisé au moins en deux parties, à savoir avec un élément transparent (32) et un élément de liaison (34), l'élément de liaison (34) étant relié à la fois à l'élément transparent (32), directement ou indirectement, et au support extérieur (20), le composant interne étant monté de façon stérile étanche dans le support extérieur et formant une fenêtre, notamment pour permettre un contrôle der processus spectral depuis l'extérieur du bioréacteur.

2. Élément composite biocompatible (10) selon la revendication 1,
- dans lequel le support extérieur (20) est réalisé en forme d'anneau, de telle sorte le support extérieur entoure en section le composant interne, et/ou
- dans lequel le support extérieur (20) est réalisé sous forme de tube, avec une première et une deuxième extrémité de tube (20p, 20d), notamment de telle sorte que le support extérieur (20) peut être raccordé avec la première extrémité de tube (20p) à la paroi du bioréacteur, et/ou
- dans lequel le support extérieur présente une collerette (22), destinée notamment au raccordement à la paroi du bioréacteur, en particulier au raccordement non séparable à la paroi du bioréacteur à usage unique, et la collerette (22) étant de préférence disposée entre la première et la deuxième extrémité de tube ou sur la première extrémité de tube (20p), et/ou
- dans lequel le support extérieur présente un filetage (24) qui est réalisé notamment sous forme de filetage extérieur, le filetage (24) étant de préférence prévu sur la première extrémité de tube (20p).

3. Élément composite biocompatible (10) selon la revendication 1 ou 2,
- dans lequel l'élément transparent (32) du composant interne (30) est réalisé sous forme d'élément transparent (32) en forme de disque.

4. Élément composite biocompatible (10) selon la revendication 1 ou 2,
- dans lequel l'élément de liaison (34) du composant interne (30) est réalisé sous forme d'élément de liaison (34) en forme de tube.

5. Élément composite biocompatible selon l'une des revendications 1 à 4,
- dans lequel l'élément de liaison (34) entoure en section l'élément transparent (32), de telle sorte que l'élément transparent (32) est monté de façon stérile étanche dans l'élément de liaison (34) et/ou est relié à l'élément de liaison (34), et que l'élément de liaison (34) est à son tour monté de façon stérile étanche dans le support extérieur (20), notamment de façon inséparable, et/ou
- dans lequel l'élément de liaison (34) entoure en section un support (33) entourant l'élément transparent (32), de telle sorte que l'élément transparent (32) est monté de façon stérile étanche dans le support (33), que le support (33) est à son tour monté de façon stérile étanche dans l'élément de liaison (34), et que l'élément de liaison (34) est à son tour monté de façon stérile étanche dans le support extérieur (20).

6. Élément composite biocompatible (10) selon l'une des revendications 1 à 4,
- dans lequel l'élément de liaison (34) est contigu côté face frontale à l'élément transparent (32) et/ou est relié côté face frontale à l'élément transparent (32), de telle sorte qu'aussi bien l'élément transparent (32) que l'élément de liaison (34) sont montés de façon stérile étanche dans le support extérieur (20), notamment de façon inséparable.

7. Élément composite biocompatible (10) selon l'une des revendications 1 à 4,
- dans lequel l'élément de liaison (34) est relié côté face frontale à l'élément transparent (32) et/ou à un support (33) entourant l'élément transparent (32), de telle sorte que l'élément transparent (32) est monté de façon stérile étanche dans le support (33), que le support (33)est à son tour relié de façon stérile étanche à l'élément de liaison (34), et que l'élément de liaison (34) est à son tour monté de façon stérile étanche dans le support extérieur (20), et/ou de telle sorte que l'élément transparent (32) est relié de façon stérile étanche à l'élément de liaison (34) et que l'élément de liaison (34) est à son tour monté de façon stérile étanche dans le support extérieur (20), et/ou
- dans lequel le composant interne (30) dépasse au moins en partie du support extérieur (20), et dépasse notamment avec l'élément transparent (32) et/ou le support (33) du support extérieur (20).

8. Élément composite biocompatible (10) selon l'une des revendications 1 à 7,
- dans lequel le composant interne (30) est monté avec une contrainte de compression dans le support extérieur (20), notamment de façon inséparable.

9. Élément composite biocompatible (10) selon l'une des revendications 1 à 7,
- dans lequel le composant interne (30) est monté de façon neutre en termes de contrainte dans le support extérieur (20), notamment de façon inséparable.

10. Élément composite biocompatible (10) selon la revendication 8 ou 9,
- dans lequel l'élément transparent (32) est monté avec une contrainte de compression dans l'élément de liaison (34), notamment de façon inséparable.

11. Élément composite biocompatible (10) selon la revendication 8 ou 9,
- dans lequel l'élément transparent (32) est relié de façon neutre en termes de contrainte à l'élément de liaison (34), notamment de façon inséparable.

12. Procédé de fabrication d'un élément composite biocompatible (10) comprenant un support extérieur (20), comportant un matériau polymère ou constitué d'un tel matériau, et un composant interne qui est réalisé en au moins deux parties, à savoir avec un élément transparent (32) et un élément de liaison (34), l'élément de liaison (34) étant relié à la fois à l'élément transparent (32), directement ou indirectement, et au support extérieur (20), le composant interne étant monté de façon stérile étanche dans le support extérieur et formant une fenêtre, le procédé comprenant :
- la préparation du support extérieur (20), comportant un matériau polymère ou constitué d'un tel matériau, et du composant interne (30),
- la réalisation d'une dilatation relative du support extérieur (20) par rapport au composant interne (30), notamment par chauffage du support extérieur ou de préférence par chauffage conjoint du support extérieur et du composant interne,
- la mise en place du composant interne (30) dans le support extérieur (20) dilaté par rapport au composant interne (30),
- la réalisation d'un retrait relatif du support extérieur (20) par rapport au composant interne (30), notamment par refroidissement du support extérieur ou de préférence par refroidissement conjoint du support extérieur et du composant interne, de telle sorte que le support extérieur (20) contient le composant interne (30) de façon stérile étanche.

13. Procédé de fabrication d'un élément composite biocompatible (10) comprenant un support extérieur (20), comportant un matériau polymère ou constitué d'un tel matériau, et un composant interne qui est réalisé en au moins deux parties, à savoir avec un élément transparent (32) et un élément de liaison (34), l'élément de liaison (34) étant relié à la fois à l'élément transparent (32), directement ou indirectement, et au support extérieur (20), le composant interne étant monté de façon stérile étanche dans le support extérieur et formant une fenêtre, le procédé comprenant :
- la préparation du composant interne (30) et d'un matériau polymère liquide pour réaliser le support extérieur, le matériau polymère formant avec le matériau ou avec un matériau du composant interne (30) de préférence un angle de mouillage qui est inférieur à 90 °,
- l'apport du matériau polymère liquide, de l'extérieur, sur le composant interne (30), notamment sur l'élément de liaison (34) et/ou sur l'élément transparent (32),
- le durcissement du matériau polymère liquide, de manière à former un support extérieur (20) recevant le composant interne (30) de façon stérile étanche.

14. Procédé selon la revendication 13, destiné à la fabrication d'un élément composite biocompatible (10), notamment selon la revendication 10, comprenant :
- la préparation d'un élément transparent (32) et d'un élément de liaison (34), notamment en métal,
- la réalisation d'une dilatation relative de l'élément de liaison (34) par rapport à l'élément transparent (32), notamment par chauffage de l'élément de liaison (34) ou de préférence par chauffage conjoint de l'élément de liaison et de l'élément transparent,
- la mise en place de l'élément transparent (32) dans l'élément de liaison (34) qui est dilaté par rapport à l'élément transparent (32),
- la réalisation d'un retrait relatif de l'élément de liaison (34) par rapport à l'élément transparent (32), notamment par refroidissement de l'élément de liaison ou de préférence par refroidissement conjoint de l'élément de liaison et de l'élément transparent, de telle sorte que l'élément de liaison (34) se lie à l'élément transparent (32) pour former le composant interne (30),
- l'exécution des étapes de procédé selon la revendication 13.

15. Procédé selon la revendication 13, destiné à la fabrication d'un élément composite biocompatible (10), notamment selon la revendication 11, comprenant :
- la préparation d'un élément transparent (32) et d'un élément de liaison (34), constitué notamment du même matériau que l'élément transparent (32),
- le rapprochement et/ou la jonction directe de l'élément de liaison (34), côté face frontale, avec l'élément transparent (32) et, de préférence le soudage au laser de l'élément de liaison (34) avec l'élément transparent (32), de telle sorte que l'élément de liaison (34) se lie à l'élément transparent (32) pour former le composant interne (30),
- l'exécution des étapes de procédé selon la revendication 13.

16. Procédé selon l'une des revendications 12 à 15, destiné à la fabrication d'un élément composite biocompatible (10), comprenant :
- la stérilisation, notamment le passage à l'autoclave, de l'élément de liaison (34) comportant le support extérieur (20) et le composant interne (30) qui y est logé de façon stérile étanche.

17. Bioréacteur, en particulier bioréacteur à usage unique, destiné à la culture de micro-organismes ou de cellules, comprenant un élément composite (10) relié en tant que pièce de raccordement à une paroi du bioréacteur, selon l'une des revendications 1 à 11,
- dans lequel le support extérieur (20) est raccordé à la paroi du bioréacteur, notamment de façon stérile étanche, et
- dans lequel le composant interne forme une fenêtre dans le bioréacteur, notamment pour permettre un contrôle de processus spectral depuis l'extérieur du bioréacteur.

18. Procédé de multiplication ou de culture de matière biologique dans un bioréacteur selon la revendication 17, englobant notamment la fabrication de produits pharmaceutiques, en particulier de produits biopharmaceutiques, comprenant un élément composite biocompatible (10) selon l'une des revendications 1 à 11, qui est relié en tant que pièce de raccordement à une paroi du bioréacteur, un contrôle de processus spectral étant effectué depuis l'extérieur du bioréacteur, en mesurant notamment une grandeur physique, chimique ou biologique depuis l'extérieur du bioréacteur, à travers la fenêtre formée.
